# EUROPEAN PATENT SPECIFICATION

(11) **EP 0 630 382 B1**
(45) Date of publication and mention of the grant of the patent: **26.02.1997**
(21) Application number: 93910157.2
(22) Date of filing: 15.04.1993
(51) Int. Cl.: C07K 5/06, A61K 38/55

(54) **LACTAM DIPEPTIDES HAVING HLE INHIBITING ACTIVITY**
Lactam Dipeptide mit HLE inhibierende Aktivität.
DIPEPTIDES DE LACTAME A ACTIVITE D'INHIBITION DE L'ELASTASE LEUCOCYTAIRE HUMAINE

(30) Priority: 16.04.1992 GB 9208380; 16.04.1992 GB 9208379; 08.07.1992 GB 9214448; 14.08.1992 GB 9217362; 14.08.1992 GB 9217363; 14.08.1992 GB 9217364
(43) Date of publication of application: 28.12.1994
(73) Proprietor: ZENECA LIMITED, London W1Y 6LN (GB)
(72) Inventor: BERSTEIN, Peter, Robert, Wallingford, PA 19086-6721 (US); THOMAS, Royston Martin, Macclesfield, Cheshire SK10 3AT (GB); WARNER, Peter, Macclesfield, Cheshire SK10 2TZ (GB); WOLANIN, Donald, John, Orange, CT 06477 (US)
(74) Representative: Smith, Stephen Collyer
(86) International application number: GB9300794
(87) International publication number: WO9321212

(56) References cited:
- EP-A- 0 369 391
- EP-A- 0 509 769
- EUROPEAN JOURNAL OF PHARMACOLOGY vol. 193, no. 2, 7 February 1991, pages 153-158, C.P. SOMMERHOFF et al.

## Description

The present invention relates to certain heterocyclic compounds, in particular, certain 1-pyridylacetamide compounds, which are inhibitors of human leukocyte elastase (HLE), also known as human neutrophil elastase (HNE), making them useful whenever such inhibition is desired, such as for research tools in pharmacological, diagnostic and related studies and in the treatment of diseases in mammals in which HLE is implicated. For example, HLE has been implicated in the pathogenesis of acute respiratory distress syndrome (ARDS), rheumatoid arthritis, atherosclerosis, pulmonary emphysema, and other inflammatory disorders, including airway inflammatory diseases characterized by increased and abnormal airway secretion such as chronic bronchitis and cystic fibrosis. Also, HLE has been implicated in certain vascular diseases and related conditions (and their therapy) in which neutrophil participation is involved or implicated, for example, in hemorrhage associated with acute non-lymphocytic leukemia, as well as in reperfusion injury associated with, for example, myocardial ischaemia and related conditions associated with coronary artery disease such as angina and infarction, cerebrovascular ischaemia such as transient ischaemic attack and stroke, peripheral occlusive vascular disease such as intermittent claudication and critical limb ischaemia, venous insufficiency such as venous hypertension, varicose veins and venous ulceration, as well as impaired reperfusion states such as those associated with reconstructive vascular surgery, thrombolysis and angioplasty. The invention also includes intermediates useful in the synthesis of these heterocyclic compounds, processes for preparing the heterocyclic compounds, pharmaceutical compositions containing such heterocyclic compounds and methods for their use.

In U.S. Patent 4,910,190, of 20 March 1990, assigned to ICI Americas Inc. (now Zeneca Inc.), there is disclosed a series of peptidoyl trifluoromethane derivatives which are HLE inhibitors.

In EP-A-509769 there is disclosed a series of 1-pyridylacetamide compounds which are HLE inhibitors.

Disclosed herein is a series of substituted 2-(2-oxo-1,2-dihydro-1-pyridyl)-N-[3,3,3-trifluoro-1-(lower alkyl)-2-oxopropyl]acetamide derivatives, which unexpectedly possess inhibitory properties against HLE, which provides the basis for the present invention.

According to the invention there is provided a Compound of the invention which is a compound of formula I (formula set out, together with other formulae referred to by Roman numerals, following the Examples) wherein:
R⁰ is (1-5C)alkyl;
R is 2,2,2-trifluoroethoxycarbonyl or 2,2,2-trifluoroethylaminocarbonyl; or
R is a sulfonyl group of formula D.W.SO₂- in which D.W-, taken together, is hydroxy, amino, di(lower alkyl)amino, 2,2,2-trifluoroethylamino, 3,3,3-trifluoropropyl, 2,2,2-trifluoroethyl or trifluoromethyl; or
W is a direct bond, imino, carbonylimino, oxycarbonylimino or iminocarbonylimino; and
D is as defined below; or
R is a group G as defined below;

The group D or G is (1-6C)alkyl, (3-6C)cycloalkyl, (3-6C)cycloalkyl-(1-3C)alkyl, aryl, aryl(1-3C)alkyl, heteroaryl or heteroaryl(1-3C)-alkyl wherein an alkyl carbon of G not bonded to the pyridone 3-amino nitrogen may bear an oxo group and wherein an aryl or heteroaryl moiety may bear one or more halogeno, nitro, methyl or trifluoromethyl groups and further wherein the group D or G may bear one or more substituents selected from a group consisting of hydroxy, lower alkoxy, lower acyloxy, COORa, CH₂COORa, CONRbRc, CH₂CONRbRC, COO(CH₂)₂NReRf, cyano, SO₂R¹, CONRdSO₂R¹, NReRf, NRgCHO, NRgCOR², NRgCOOR², NRhCQNRiRj, NRkSO₂R³, SO₂NRlRm, SO₂NRnCOR⁴ and P(O)(ORa)₂ in which
Q is oxygen or sulfur;
Ra-Rn are independently hydrogen, benzyl or lower alkyl; or, independently, a group NRbRc, NReRf, NRiRj or NRlRm is a cyclic radical selected from a group consisting of 1-pyrrolidinyl, piperidino, morpholino or 1-piperazinyl which may bear a lower alkyl substituent at the 4-position; or, independently, a group NReRf is a cyclic radical selected from a group consisting of 2-pyrrolidinon-1-yl, succinimido, oxazolidin-2-on-3-yl, 2-benzoxazolinon-3-yl, phthalimido and cis-hexahydrophthalimido; and
R¹-R⁴ are independently trifluoromethyl, (1-6C)alkyl, (3-6C)cycloalkyl, aryl or heteroaryl in which the aryl or heteroaryl may bear one or more substituents selected from a group consisting of lower alkyl, hydroxy, lower alkoxy, halogeno or trifluoromethyl;

Each of R⁵ and R⁶ is, independently, hydrogen or lower alkyl; or

One of R⁵ and R⁶ is hydrogen or methyl and the other of R⁵ and R⁶ is a radical of formula B.Y- in which
B is aryl or heteroaryl, which aryl or heteroaryl independently may bear one or more of the substituents defined for D or G or an aryl or heteroaryl moiety thereof;
Y is a direct bond, methylene, ethylene or trans-vinylene; and
provided that no aliphatic carbon is bonded to more than one nitrogen or oxygen, except as part of a cyclic ketal or where the nitrogen bears a carbonyl group; or,
for a compound of formula I which is acidic or basic, a pharmaceutically acceptable salt thereof.

In this specification, the following definitions are used, unless otherwise described: Halogeno is fluoro, chloro, bromo or iodo. Alkyl, alkoxy, etc. denote both straight and branched groups; but reference to an individual radical such "propyl" embraces only the straight chain ("normal") radical, a branched chain isomer such as "isopropyl" being specifically referred to. Lower alkyl and lower alkoxy refer to radicals containing one to about four carbon atoms. Lower acyloxy refers to a radical containing one to about five carbon atoms. Aryl denotes a phenyl radical or an ortho-fused bicyclic carbocyclic radical having about nine to ten ring atoms in which at least one ring is aromatic. Heteroaryl encompasses a radical attached via a ring carbon of a monocyclic aromatic ring containing five or six ring atoms consisting of carbon and one to four heteroatoms selected from the group consisting of oxygen, sulfur and nitrogen, as well as a radical of an ortho-fused bicyclic heterocycle of about eight to ten ring atoms derived therefrom, particularly a benz-derivative or one derived by fusing a propenylene, trimethylene or tetramethylene diradical thereto, as well as a stable N-oxide thereof.

It will be appreciated that, owing to the asymmetrically substituted carbon atom at the chiral center indicated by "*" in formula I, a compound of formula I may exist in, and be isolated in, optically active and racemic forms. If a compound of formula I contains an additional chiral element, such compound of formula I may exist in, and be isolated in, the form of a diastereomeric mixture or as a single diastereomer. It is to be understood that the present invention encompasses a compound of formula I as a mixture of diastereomers, as well as in the form of an individual diastereomer, and that the present invention encompasses a compound of formula I as a mixture of enantiomers, as well as in the form of an individual enantiomer. Vhen R⁰ is isopropyl, a compound of formula I may be viewed as an alanyl trifluoromethane derivative. In general, a compound of formula I having the (S)-configuration at the chiral center indicated by "*" which corresponds to the L-alanyl configuration, is preferred. Accordingly, it may be preferred to use the compound of formula I in a form which is characterized as containing, for example, at least 95%, 98% or 99% enantiomeric excess (ee) of the (S)-form. However, owing to the interconvertability of the (S)-isomer and the (R)-isomer by the facile epimerization of the chiral center indicated by "*" in formula I, it may be preferred to utilize a compound of formula I as a mixture of the (S)- and (R)-isomers at the center indicated by "*" in formula I.

As will be appreciated by those skilled in the art, a trifluoromethyl ketone of formula I can exist as a solvate, particularly a hydrate; and such a solvate of a compound of formula I is encompassed by the present invention.

A compound of formula I may exhibit polymorphism. The compound may form solvates in addition to a ketone solvate mentioned above. A compound may exist in more than one tautomeric form. It is to be understood, therefore, that the present invention encompasses any racemic or optically-active form, any polymorphic form, any tautomer or any solvate, or any mixture thereof, which form possesses inhibitory properties against HLE, it being well known in the art how to prepare optically active forms (for example, by resolution of the racemic form or by synthesis from optically-active starting materials) and how to determine the inhibitory properties against HLE by the standard tests described hereinafter.

It is preferred that the radicals R⁰, R, R⁵ and R⁶ not contain nor introduce an additional element of chirality into the molecule beyond the chiral center indicated by "*" in formula I.

Particular values are listed below for radicals, substituents and ranges for illustration only and they do not exclude other defined values or other values within defined ranges for the radicals and substituents.

A particular value for R⁰ is ethyl or isopropyl.

A particular value for D.W-, taken together, is amino, 2,2,2-trifluoroethylamino or 2,2,2-trifluoroethyl.

A particular value for W is a direct bond or imino.

A particular value for G is (1-3C)alkyl, aryl(1-C)alkyl or heteroaryl(1-2C)alkyl which may bear one or more substituents as defined above for G or a part thereof.

A particular value of (1-6C)alkyl is methyl, ethyl, propyl, isopropyl, butyl, isobutyl, t-butyl, pentyl, 3-methylbutyl, 1-ethylpropyl, hexyl or 4-methylpentyl. A particular value of (3-6C)cycloalkyl is cyclopropyl, cyclopentyl or cyclohexyl. A particular value for the (1-3C)alkyl portion of (3-6C)cycloalkyl(1-3C)alkyl, aryl(1-3C)alkyl or heteroaryl(1-3C)alkyl is methylene, ethylene or trimethylene. A particular value for aryl is phenyl, indenyl or naphthyl. A particular value for heteroaryl is furyl, imidazolyl, tetrazolyl, pyridyl (or its N-oxide), thienyl, pyrimidinyl (or its N-oxide), indolyl or quinolinyl (or its N-oxide). A particular value for lower alkyl is methyl, ethyl, propyl, isopropyl, butyl, isobutyl or t-butyl. A particular value for lower acyloxy is acetoxy. A particular value for lower alkoxy is methoxy, ethoxy, propoxy, isoproxy or t-butoxy. A particular value for halogeno is bromo, chloro or fluoro.

A particular value for COORa is carboxy or methoxycarbonyl. A particular value for NRgCHO is formylamino. A particular value for NRgCOR² is acetylamino or trifluoroacetylamino. A particular value of CONRdSO₂R¹ is N-phenylsulfonylcarbamoyl or N-(4-chlorophenylsulfonyl)-carbamoyl.

A more particular value for R⁰ is isopropyl. A more particular value for D is methyl, ethyl, isopropyl, tert-butyl, cyclohexyl, phenyl, benzyl, phenethyl, pyridyl, thienyl, 5-tetrazolyl, thiazolyl, quinolinyl, pyridylmethyl, thenyl, 5-tetrazolylmethyl, 2-(pyridyl)ethyl, 2-(thienyl)ethyl or 2-(thiazolyl)ethyl wherein the phenyl or heteroaryl group may bear one or two halogeno or methyl groups and further wherein the group D may bear a substituent selected from hydroxy, methoxy, t-butoxy, acetoxy, pivaloyloxy, carboxy, methoxycarbonyl, ethoxycarbonyl, carbamoyl, dimethylcarbamoyl, 2-(dimethylamino)ethoxycarbonyl, cyano, methylsulfonyl, phenylsulfonyl, N-methylsulfonylcarbamoyl, N-phenylsulfonylcarbamoyl, N-(4-chlorophenylsulfonyl)carbamoyl, methylsulfonylamino, amino, dimethylamino, oxazolidin-2-on-3-yl, acetylamino, trifluoroacetylamino, ureido, methylsulfonyl, sulfamoyl, dimethylphosphoryl or diethylphosphoryl. A more particular value for G is methyl, ethyl, benzyl, phenethyl, pyridyl, pyridylmethyl, thenyl, 5-tetrazolylmethyl, or 2-(pyridyl)ethyl, wherein an alkyl carbon may bear an oxo group and wherein the phenyl or heteroaryl group may bear one or two halogeno or methyl groups and further wherein the group G may bear a substituent selected from hydroxy, methoxy, acetoxy, carboxy, methoxycarbonyl, ethoxycarbonyl, carbamoyl, dimethylcarbamoyl, phenylcarbamoyl, pyridylcarbamoyl, methylsulfonylamino, amino, dimethylamino, acetylamino, nicotinoylamino, or trifluoroacetylamino.

A particular value for R is, for example, sulfo, aminosulfonyl, dimethylaminosulfonyl, 2,2,2-trifluoroethylaminosulfonyl, 2,2,2-trifluoroethylsulfonyl, trifluoromethylsulfonyl, methylsulfonyl (which may bear a methoxycarbonyl, carboxy or ethylsulfonyl substituent), methylaminosulfonyl, isopropylamino-sulfonyl, butylsulfonyl, butylaminosulfonyl, tert-butylaminosulfonyl, cyclohexylaminosulfonyl, phenylsulfonyl (in which the phenyl may bear a chloro, nitro, amino, acetylamino, trifluoroacetylamino, methoxy, carboxy, N-(4-chlorophenylsulfonyl)carbamoyl, or methylsulfonylamino substituent at the 3- or 4-position), anilino, pyridylsulfonyl, quinolinylsulfonyl, benzylsulfonyl (in which the phenyl ring may bear a nitro or amino substituent at the 3- or 4-position), pyridylmethylsulfonyl, 2-(pyridyl)ethylsulfonyl or benzylaminosulfonyl.

One particular group of compounds of formula I is one in which R⁰ and R have any of the values defined above, R⁵ is hydrogen and R⁶ is hydrogen.

Another particular group of compounds of formula I is one in which R⁰ and R have any of the values defined above, R⁵ is benzyl, the phenyl ring of which may bear a 3-fluoro, 4-fluoro, 4-trifluoromethyl, 4-methoxycarbonyl, 3-acetoxy, 3-hydroxy, 3-pivaloyloxy, 4-hydroxy, 4-pivaloyloxy, 3-trifluoroacetylamino or 3-amino substituent, and R⁶ is hydrogen.

A further particular group of compounds of formula I is one in which R⁰ and R have any of the values defined above, R⁵ is hydrogen, and R⁶ is 2-furyl, 2-thienyl, 3-pyridyl or phenyl in which the phenyl may bear one or two halogeno, trifluoromethyl, methyl, hydroxy, methoxy, tert-butoxy, methoxycarbonyl or carboxy substituents; and, more particularly, R⁶ is phenyl, 4-fluorophenyl or 2-thienyl.

Specific compounds of formula I are described in the accompanying Examples. Of these, compounds of particular interest, along with their pharmaceutically acceptable salts, include those described in Examples 21, 45, 46 and 57.

A pharmaceutically acceptable salt of an acidic compound of formula I is one made with a base which affords a pharmaceutically acceptable cation, which includes alkalai metal salts (especially lithium, sodium and potassium), alkaline earth metal salts (especially calcium and magnesium), aluminum salts and ammonium salts, as well as salts made from appropriate organic bases such as triethylamine, morpholine, piperidine and triethanol amine. A pharmaceutically acceptable salt of a basic compound of formula I includes an acid-addition salt made with an acid which provides a pharmaceutically acceptable anion, including for example, a strong acid such as hydrochloric, sulfuric or phosphoric acid.

A compound of formula I may be made by processes which include processes known in the chemical art for the production of structurally analogous heterocyclic and peptidic compounds. Such processes and intermediates for the manufacture of a compound of formula I as defined above are provided as further features of the invention and are illustrated by the following procedures in which the meanings of generic radicals are as defined above:
(A) Oxidizing a corresponding alcohol of formula II. It will be recognized that protection of the pyridone 3-amino substituent prior to oxidation and removal of the protecting group after oxidation may be required or preferred if the amino group is not stable to the oxidation conditions employed. A convenient method may be the use of excess dimethyl sulfoxide and a water soluble carbodimide, with dichloroacetic acid as a catalyst, in a inert solvent such as toluene at about room temperature, for example as described in Example 1. Other methods which may be useful include the use of alkaline aqueous potassium permanganate solution; the use of oxalyl chloride, dimethyl sulfoxide and a tertiary amine; the use of acetic anhydride and dimethyl sulfoxide; the use of chromium trioxide pyridine complex in methylene chloride; and the use of a hypervalent iodine reagent, such as a periodinane, for example 1,1,1-triacetoxy-2,1-benzoxidol-3(3H)-one with trifluoroacetic acid in dichloromethane.
(B) For a compound of formula I which bears a hydroxy substituent on an aryl or heteroaryl group, cleaving the alkyl ether or acyloxy ester of a corresponding compound of formula I which bears a lower alkoxy or lower acyloxy substituent on an aryl or heteroaryl group. Convenient methods include, for example, the cleavage of a methoxy group using boron tribromide and the cleavage of a t-butoxy group using trifluoroacetic acid for an alkyl ether, and the acidic or alkaline hydrolysis of an acyloxy group.
(C) For a compound of formula I wherein R is 2,2,2-trifluoroethoxycarbonyl or 2,2,2-trifluoroethylaminocarbonyl, acylation of a corresponding amine of formula V with 2,2,2-trifluoroethyl chloroformate or 2,2,2-trifluoroethyl isocyanate. The acylation is conveniently carried out in an inert solvent or diluent, such as dichloromethane, tetrahydrofuran or toluene, at about ambient temperature, using an organic base such as, for example, triethylamine or pyridine, or an inorganic base, such as sodium or potassium carbonate, as an acid acceptor when the chloroformate is used. When the isocyanate is used, it may be preferred to use a catalyst, such as for example, cuprous chloride.
(D) For a compound of formula I wherein R is a sulfonyl group of formula D.W.SO²-, sulfonylation of a corresponding amine of formula V with a corresponding sulfonic acid of formula D.W.SO₂.OH, or an activated derivative thereof, such as an acid halide, particularly a sulfonyl (or sulfamoyl) chloride of formula D.W.SO₂.Cl. The sulfonylation is conveniently carried out in an inert solvent or diluent, such as dichloromethane, tetrahydrofuran or toluene, at about ambient temperature, using an organic base such as, for example, triethylamine or pyridine, or an inorganic base, such as sodium or potassium carbonate, as an acid acceptor. If a sulfonyl chloride is not commercially available, it may be obtained by a conventional method.
(E) For a compound of formula I wherein R is a group G, substitution of the group X of a corresponding compound of formula G-X, wherein X is a conventional leaving group, such as for example halogeno, methylsulfonyloxy, trifluoromethylsulfonyloxy or diazonium, with a corresponding amine of formula V, optionally using a conventional catalyst.
(F) For a compound of formula I which bears a group of formula COORa in which Ra is hydrogen (a carboxy group), decomposing the ester group of a corresponding ester made with a conveniently removed acid protecting group, for example a corresponding compound of formula I in which Ra is not hydrogen. The decomposition may be carried out using any one of the variety of procedures well known in organic chemistry, for example basic hydrolysis using lithium or sodium hydroxide, or by hydrogenolysis of a benzyl ester.
(G) Removal by using a conventional method of the nitrogen protecting group of a corresponding compound bearing a conventional nitrogen protecting group to afford a corresponding compound of formula I which contains an amino N-H residue; particularly for a compound of formula I wherein R is G, the removal of an activating/protecting group Rx from a corresponding compound of formula Vb. Rx is a group which protects and activates a primary amino group for substitution, such as for example benzyloxycarbonyl or trifluoroacetyl. Conventional methods include, for example, removal of a benzyloxycarbonyl group by hydrogenolysis, removal of a benzyloxycarbonyl or tert-butoxycarbonyl group by treatment with a strong acid, for example with trifluoromethanesulfonic acid in an inert solvent such as dichloromethane, or basic hydrolysis of a trifluoroacetyl group.
(H) For a compound of formula I bearing a moiety of formula COORa, CH₂COORa, CONRbRc, CH₂CONRbRc, COO(CH₂)₂NReRf or CONRdSO₂R¹, acylation of a corresponding compound of formula BORa, HNRbRc, HO(CH2)₂NReRf or HNRdSO₂R¹ with a corresponding acid of formula I bearing a moiety of formula COORa in which Ra is hydrogen, or an activated derivative thereof.
(I) For a compound of formula I bearing a lower acyloxy group or a group of formula NRgCHO, NRgCOR², WgCOOR², NRhCQNRiRj or NRkSO₂R³, acylation or sulfonylation of a corresponding compound of formula I bearing a hydroxy group or an amino group of formula NHRg, NHRh or NHRk (i.e. an amino group of formula NReRf is which Re is hydrogen and Rf is Rg, Rh or Rk) with an activated derivative of a corresponding acid of formula HOCHO, HOCOR², HOCOOR², HOCQNRiRj (including an isocyanate or isothiocyanate) or HOSO₂R³, respectively, using a conventional method.
(J) For a compound of formula I which bears a heteroaryl N-oxide group, oxidation of a corresponding compound of formula I which bears a heteroaryl group using a conventional oxidant, such as for example dioxirane in acetone.
(K) For a compound of formula I which bears a primary amino group, reduction of a corresponding compound bearing a nitro group using a conventional reducing method, such as for example, hydrogenation over a palladium catalyst, or reduction with tin(II) chloride.

Whereafter, for any of the above procedures, when a pharmaceutically acceptable salt of an acidic or basic compound of formula I is required, it may be obtained by reacting the acidic or basic form of such a compound of formula I with a base or acid affording a physiologically acceptable counterion or by any other conventional procedure.

If not commercially available, the necessary starting materials for the above procedures may be made by procedures which are selected from standard techniques of heterocyclic chemistry and peptide chemistry, techniques which are analogous to the synthesis of known, structurally similar compounds, and techniques which are analogous to the above described procedures or the procedures described in the Examples. For uniformity and clarity, compounds herein are represented as the 2-pyridone, rather than the 2-hydroxypyridine, tautomers.

As will be clear to one skilled in the art, a variety of sequences is available for preparation of the starting materials. According to one of the available routes, a key intermediate pyrid-2-one-3-carboxylic acid of formula III may be prepared as shown in Scheme I (set out, together with other Schemes, following Examples) and as described in the Examples. In the Schemes, CBZ represents a benzyloxycarbonyl group.

In general, in a formal sense, a ketone of formula R⁵.CH₂.CO.R⁶ may be formylated, then cyclized with cyanoacetamide to afford a pyrid-2-one-3-carbonitrile of formula IV. Thus (Cyclization Method A) the ketone may be formylated with dimethylformamide dimethyl acetal in acetonitrile then cyclized with cyanoacetamide, using sodium methoxide in dimethylformamide. Alternatively, (Cyclization Method B) the ketone may be formylated using sodium methoxide and ethyl formate in tetrahydrofuran or ether, distilling the solvent, dissolving the resulting salt in water, adding acetic acid to pH 9, and heating with cyanoacetamide at 90 °C to achieve the cyclization. As a further varation, (Cyclization Method C) the salt resulting from formylation with sodium methoxide and ethyl formate, followed by removal of the solvent, may be cyclized with cyanoacetamide by heating an aqueous solution with piperidine acetate as a catalyst. Where more than one product is possible from the cyclization reaction, the product selectivity may be controlled by the cyclization (and formylation) method chosen. For example, cyclization of phenylacetone by Cyclization Method A affords 6-methyl-5-phenylpyrid-2-one-3-carbonitrile; but cyclization of phenylacetone by Cyclization Method C affords 6-benzylpyrid-2-one-3-carbonitrile. Hydrolysis of the cyano group of a compound of formula IV, for example by heating with 48% hydrobromic acid in acetic acid (Hydrolysis Method A,) or with sodium hydroxide solution in a pressure vessel (Hydrolysis Method B) affords a corresponding carboxy derivative of formula III. For a compound in which R⁶ is B.Y- and Y is ethylene or trans-vinylene, it may be preferred to proceed via an alternative route to an acid of formula III. Thus, cyclization of a ketone of formula R⁵.CH₂.CO.CH₃ affords a 6-methyl pyridone derivative of formula IVa, for example, cyclizing acetone by Cyclization Method C. Bis-metallation, followed by alkylation with a reagent of, for example, formula B.CH₂.Br affords a corresponding nitrile of formula IV in which Y is ethylene. Alternatively, bis-metallation of a 6-methyl pyridone of formula IVa, followed by condensation with an aldehyde of formula B.CHO, affords a pyrid-2-one-3-carbonitrile of formula IVb which may be converted by acid hydrolysis and dehydration into a corresponding pyride-2-one-3-carboxylic acid of formula III in which Y is trans-vinylene.

An acid of formula III may be converted into a corresponding isocyanate of formula VI by a conventional method, for example by using diphenylphosphoryl azide in an inert solvent. Conveniently, the isocyanate is not isolated, but is converted into a benzyl urethane of formula VII as also is shown in Scheme I. (An isocyanate of formula VI may undergo intramolecular cyclization to the oxygen at the pyridone 2-position, thereby forming a corresponding cyclic carbamate, which carbamate similarly may afford a corresponding compound of formula VII.)

Elaboration of a substituted amino pyridone of formula VII into a corresponding intermediate alcohol of formula II or a corresponding intermediate amine of formula V or protected amine formula Vb may be carried out as outlined in Scheme II. Alkylation of a compound of formula VII with an iodoacetamide derivative, for a compound in which R⁰ is isopropyl, affords a 1-substituted pyridone of formula VIII, wherein Rp represents an alcohol protecting group, conveniently tert-butyldimethylsilyl. (The corresponding 2-alkoxypyridine resulting from 0-alkylation is also obtained. When R⁶ is subject to hindered rotation, for example when R⁵ is methyl and R⁶ is phenyl, or, for example, when R⁵ is hydrogen and R⁶ is 2-chlorophenyl, the ratio of N-alkylated product to O-alkylated product is increased.) The benzyloxycarbonyl group of a compound of formula VIII may be removed by a conventional method, for example by hydrogenolysis, to afford a corresponding 3-amino pyridone of formula IX.

For a compound of formula X wherein R is 2,2,2-trifluoroethoxy carbonyl or 2,2,2-trifluoroethylaminocarbonyl, a 3-amino pyridone of formula IX may be acylated by using a method similar to one described above in process (C) to afford a corresponding pyridone of formula X. For a compound of formula X wherein R is a sulfonyl group of formula D.W.SO₂-, a 3-amino pyridone of formula IX may be sulfonylated by using a convention method to afford a corresponding pyridone of formula X. Conventional sulfonylation methods include those described above in process (D) for the sulfonylation of an amine of formula V. (Should a portion of bis-sulfonylated product be obtained, treatment with aqueous base at an elevated temperature may be used to remove the more labile second sulfonyl group at a convenient stage in the synthesis; see for example Example 6, parts a.-b.) For a compound of formula X wherein R is a group G, a 3-amino pyridone of formula IX may be subjected to a conventional substitution reaction similar to one described above in process (E) to afford a corresponding pyridone of formula X.

Removal of a tert-butyldimethylsilyl group from a compound of formula X in which Rp is tert-butyldimethylsilyl to provide a corresponding alcohol of formula II may be carried out using tetrabutylammonium fluoride in an inert solvent; it may be preferred to use acetic acid to buffer the reaction conditions.

An alternative order of steps to convert a protected compound of formula VIII into a corresponding alcohol of formula II can be used as well. Thus, removal of the alcohol protecting group of a compound of formula VIII affords the corresponding alcohol of formula VIIIa. Deprotection of the amino group of a compound of formula VIIIa affords a corresponding amino alcohol of formula XXVII (see Scheme IV for formula XXVII) which can be converted into a corresponding alcohol of formula II using a conventional procedure.

A different route which obviates the need for an alcohol deprotection step is also shown in Scheme II. Thus, a pyridone of formula VII may be alkylated, for example with ethyl or t-butyl iodoacetate, to afford a corresponding ester of formula XI, wherein Rq is a conveniently removable acid protecting group, for example ethyl or t-butyl. Removal of the acid protecting group of an ester of formula XI by a conventional method, for example by base catalyzed hydrolysis or by acid catalyzed elimination, affords a corresponding acid of formula XII. An acid of formula XII may be coupled with the requisite amino alcohol for example, with 3-amino-1,1,1-trifluoro-4-methyl-2-pentanol, to afford a corresponding alcohol of formula VIIIa.

An alternative route for the preparation of an intermediate acid of formula XII, beginning with a ketone of formula R⁵.CH₂.CO.R⁶ and involving a novel pyridone synthesis, which may be a preferred route, is described in European patent appn., pubn. no. 509 769 (see page 30 hereinafter) for the conversion of acetophenone into 3-benzyloxycarbonylamino-2-oxo-6-phenyl-1,2-dihydro-1-pyridylacetic acid. The coupling to provide the corresponding alcohol of formula VIIIa, 2-(3-benzyloxycarbonylamino-2-oxo-6-phenyl-1,2-dihydro-1-pyridyl)-N-(3,3,3-trifluoro-2-hydroxy-1-isopropylpropyl)acetamide, is also described therein.

Oxidation of an alcohol of formula VIIIa, using a method similar to one described in process (A) for oxidation of an alcohol of formula II, affords a corresponding ketone of formula VIIIb. Removal of the nitrogen protecting group of a ketone of formula VIIIb by hydrogenolysis or by treatment with a strong acid, affords a corresponding starting material amine of formula V.

A preferred method for introducing the substituent R when it is a group G, particularly when it is an alkyl or substituted alkyl group, is by the use of a corresponding compound in which the pyridone 3-amino substituent bears an activating/protecting group of formula Rx, for example, benzyloxycarbonyl or trifluoroacetyl. Thus, acylation of a compound of formula V with trifluoroacetic anhydride affords a corresponding compound of formula Va in which Rx is trifluoroacetyl, which compound may be prepared by an alternative order of steps via the corresponding compound of formula IX and 3-trifluoroacetylaminopyridones analogous to compounds of formulae VIII and VIIIa. It will be noted that a compound of formula VIIIb is, itself, a corresponding compound of formula Va in which Rx is benzyloxycarbonyl. Alkylation, using a corresponding reagent of formula G.X in which G is alkyl or substituted alkyl, then provides a corresponding intermediate of formula Vb, for example as described in Example 53.a.

Synthesis routes involving a cross coupling reaction to introduce a substituent R⁵ into intermediate compounds are outlined in Scheme III. These routes may be preferred when R⁵ has the value B.Y- and Y is methylene, ethylene or trans-vinylene. Thus, a pyridone of formula VII in which R⁵ is hydrogen may be converted into a corresponding 5-iodo pyridone of formula XXI by treatment with an iodinating agent, for example N-iodosuccinimide. An appropriate halide, for example a bromide of formula B.CH₂.Br, may be converted into a corresponding organozinc reagent, for example B.CH₂.Zn.Br, by treatment with zinc dust in tetrahydrofuran, and cross-coupled with an iodide of formula XXI using a palladium catalyst, such as dichloro[1,1'-bis(diphenylphosphino)ferrocene]palladium(II) to afford a corresponding compound of formula VII in which R⁵ is B.Y- and Y is methylene. A similar cross coupling utilizing a bromide of formula B.Y.Br in which Y is trans-vinylene may be useful to convert an iodide of formula XXI into a corresponding compound of formula VII in which R⁵ is B.Y- and Y is trans-vinylene. At a convenient point in a synthesis, a compound in which R⁵ is B.Y- and Y is trans-vinylene may be hydrogenated to afford a corresponding compound in which R⁵ is B.Y- and Y is ethylene.

Alternatively, an iodide of formula XXI may be alkylated to afford a corresponding iodide of formula XXII or XXIII which may be further cross coupled as described above to provide a corresponding compound of formula VIII or XI.

Alternative synthesis routes in which a 3-nitro pyridone serves as a precursor to a 3-amino pyridone are outlined in Scheme IV. They may be particularly useful when the 3-nitro derivative is readily available, such as when R⁵ and R⁶ are hydrogen. Alternatively, beginning with a ketone of formula R⁵.CH₂.CO.R⁶, the corresponding 3-nitropyridone may be prepared in a manner analogous to Cyclization Method A by heating a dimethylformamide solution of the ammonium salt of nitroacetamide (prepared according to J. Org. Chem. (1958), 23, 113-114) with the product isolated from treatment of the ketone with dimethylformamide dimethyl acetal in acetonitrile.

Direct reduction of the nitro group, followed by protection of the amine obtained with a benzyloxycarbonyl group provides a pyridone of formula VII, which may be converted into a corresponding intermediate of formula II, V or Vb using a route outlined in Scheme II. Alternatively, for a compound in which R is an acyl or sulfonyl group, direct reduction of the nitro group may be followed by acylation or sulfonylation of the amine obtained to provide a pyridone of formula VIIb, which may be converted into a corresponding intermediate of formula II using a route similar to one outlined in Scheme II for a compound of formula VII.

Using a different order of steps, the 3-nitro pyridone may be alkylated first to provide an ester of formula XXIV. The ester of formula XXIV may be converted into the corresponding acid of formula XXV. The acid of formula XXV also may be obtained by allylation of the starting 3-nitro pyridone, followed by oxidative cleavage of the 1-allyl group using potassium permanganate. By coupling with the appropriate amino alcohol, an acid of formula XXV may be converted into a nitro alcohol of formula XXVI. A nitro alcohol of formula XXVI may be reduced to afford a corresponding 3-amino pyridone of formula XXVII which may be converted into a corresponding intermediate alcohol of formula II using one of the methods described above. In addition, a nitro alcohol of formula XXVI may be oxidized to a corresponding nitro ketone of formula XXVIII. Reduction of the nitro group of a nitro ketone of formula XXVIII affords an intermediate amine of formula V.

An analogous route from a nitro compound of formula XXIV involves first reducing the nitro group to afford a corresponding amino compound of formula XXIX. Protection of a compound of formula XXIX with a benzyloxycarbonyl group affords a compound of formula XI, which may be further converted into a corresponding compound of formula II, V or Vb using a method described in Scheme II. Alternatively, for a compound in which R is an acyl or sulfonyl group, acylation or sulfonylation of a compound of formula XXIX affords a compound of formula XIb, which may be further converted into a corresponding compound of formula II using a similar method to that described in Scheme II for a compound of formula XI, that is, conversion into a corresponding acid of formula XIIb, followed by coupling with a requisite amino alcohol.

For a compound in which R is is a group G, it will be clear that the methodology described above using an activating/protecting group of formula Rx to introduce the substituent R on the pyridone 3-amino group may be utilized analogously at any convenient stage of a synthetic scheme.

The trifluoromethyl amino alcohols required for the synthesis routes described above may be prepared by known routes. For example, 3-amino-1,1,1-trifluoro-4-methyl-2-pentanol (as its hydrochloride salt) conveniently may be obtained as described in U.S. Patent 4,910,190 in Example 4 (as a single diastereomer) or Example 6 (as a single enantiomer of a single diastereomer). If it is desired to carry out a chiral synthesis of a compound of formula I, using the single enantiomer in a substantially enantiomerically pure form and using methods and conditions which avoid epimerization at the center indicated by "*" in formula I provide such a synthesis.

It may be desired optionally to use a protecting group during all or portions of the above described processes; the protecting group then may be removed when the final compound or a required starting material is to be formed. As will be clear to one skilled in the art, the order of steps in the sequences leading to the starting materials and products of the invention may be altered if appropriate considerations relative to coupling methods, racemization, deprotection methods, etc. are followed.

The utility of a compound of the invention or a pharmaceutically acceptable salt thereof (hereinafter, collectively referred to as a "Compound") may be demonstrated by standard tests and clinical studies, including those described below.

### Inhibition Measurements:

The potency of a Compound to act as an inhibitor of human leukocyte elastase (HLE) on the low molecular weight peptide substrate methoxy-succinyl-alanyl-alanyl-prolyl-valine-p-nitroanilide is determined as described in U.S. Patent 4,910,190. The potency of an inhibitor is evaluated by obtaining a kinetic determination of the dissociation constant, Kᵢ, of the complex formed from the interaction of the inhibitor with HLE. If a Compound is found to be a "slow-binding" inhibitor of HLE, special methods of analysis to accurately determine Kᵢ values for the inhibition of HLE are carried out as described in U.S. Patent 4,910,190. In general, the Kᵢ values for Compounds of the invention which were tested are generally on the order of 10⁻⁷ M or much less.

### Acute Lung Injury Model:

Animal models of emphysema include intratracheal (i.t.) administration of an elastolytic protease to cause a slowly progressive, destructive lesion of the lung. These lesions are normally evaluated a few weeks to a few months after the initial insult. However, these proteases also induce a lesion that is evident in the first few hours. The early lesion is first hemorrhagic, progresses to an inflammatory lesion by the end of the first 24 hours and resolves in the first week post insult. To take advantage of this early lesion, the following model (described in Villiams, et al., American Review of Respiratory Diseases (1991), 144, 875-883) was used.

Hamsters are first lightly anesthetized with Brevital. Phosphate buffered saline (PBS) pH 7.4, either alone or containing human leukocyte elastase (HLE), is then administered directly into the trachea. Twenty-four hours later the animals are killed and the lungs removed and carefully trimmed of extraneous tissue. Following determination of wet lung weight, the lungs are lavaged with PBS and total lavagable red and white cells recovered are determined. The values for wet lung weights, total lavagable red cells and total lavagable white cells are elevated in a dose-dependent manner following administration of HLE. Compounds that are effective elastase inhibitors can prevent or diminish the severity of the enzyme-induced lesion resulting in lower wet lung weight and reduced values for total lavagable cells, both red and white, relative to administration of HLE alone. Compounds can be evaluated by administering them intratracheally as solutions or suspensions in PBS, either with or at various times prior to the HLE challenge (400 µg), or by dosing them intravenously or orally as solutions at various times prior to the HLE challenge (100 µg) to determine their utility in preventing an HLE lesion. A solution of a Compound is conveniently prepared using 10% polyethylene glycol 400/PBS or 10% polyethylene glycol 400/water. For a Compound which is acidic or basic, base (e.g. sodium hydroxide solution) or acid (e.g. hydrochloric acid) may be added as indicated to achieve solution. Compounds of this invention produced statistically significant reductions in wet lung weight and total lavagable cells relative to HLE alone.

### Acute Hemorrhagic Assay:

This assay relies on monitoring only the amount of hemorrhage in the lung following intratracheal administration of human neutrophil elastase (HNE). Hemorrhage is quantified by disrupting erythrocytes recovered in lung lavage fluid and comparing that to dilutions of whole hamster blood. The screening protocol, similar to that described in Fletcher et al., American Review of Respiratory Disease (1990), 141, 672-677, is as follows. Compounds demonstrated to be HNE inhibitors in vitro are conveniently prepared for dosing as described above for the Acute Lung Injury Model. The compounds are then dosed by mouth to male Syrian hamsters at a fixed time, such as 30 or 90 min, prior to intratracheal administration of 50 µg/animal of HNE in 300 µL phosphate buffered saline (PBS) pH 7.4. Four hours after enzyme administration, the animals are killed with an overdose of pentobarbital sodium, the thorax opened and the lungs and trachea removed. The excised lungs are lavaged with three changes of 2 mL normal saline via a tracheal cannula. The recovered lavages are pooled, the volumes (about 5 mL) are recorded and the lavages stored at 4 °C until assayed. For calculation of the amount of blood in each sample, the thawed lavages and a sample of whole hamster blood are sonicated to disrupt erythrocytes and appropriately diluted into individual wells of a 96-well microtiter plate. The optical densities (OD) of the disrupted lavages and blood samples are determined at 405 nm. The (µL blood equivalents) / (mL lavage) are determined by comparing the OD of the test samples with the OD of the standard curve prepared from whole hamster blood. The total µL equivalents of blood recovered is determined by multiplying recovered lavage volume by the (µL blood equivalents) / (mL lavage) for each sample. Results are reported as % inhibition of hemorrhage with respect to PBS treated controls when the test compound is given at a specified dose and time prior to administration of HNE.

No overt toxicity was observed when Compounds of the invention were administered in the above in vivo tests.

It will be appreciated that the implications of a Compound's activity in the Acute Lung Injury Model or Acute Hemorrhagic Assay are not limited to emphysema, but, rather, that the test provides evidence of general in vivo inhibition of HLE.

Compounds of the present invention which were tested exhibited activity in at least one of the tests described above under Inhibition Measurement, Acute Lung Injury Model and Acute Hemorrhagic Assay. It should be noted that, as would be expected in comparison of in vitro and in vivo results, there was not always a direct correlation between the activities of the compounds measured as Kᵢ values in the Inhibition Measurement test and the reduced values for total lavagable cells and wet lung weights relative to the administration of HLE alone obtained in the Acute Lung Injury Model test or inhibition of hemorrhage in the Acute Hemorragic Assay.

According to a further feature of the invention, there is provided a pharmaceutical composition comprising a pharmaceutically effective amount of a Compound and a pharmaceutically acceptable diluent or carrier. As noted above, another feature of the invention is a method of using a Compound of the invention in the treatment of a disease or condition in a mammal, especially a human, in which HLE is implicated.

A Compound of the present invention may be administered to a warm-blooded animal, particularly a human, in need thereof for treatment of a disease in which HLE is implicated, in the form of a conventional pharmaceutical composition, for example as generally disclosed in U.S. Patent 4,910,190. The preferred mode of administration may be via a powdered or liquid aerosol. In a powdered aerosol, a Compound of the invention may be administered in the same manner as cromolyn sodium via a 'Spinhaler' (a trademark) turbo-inhaler device obtained from Fisons Corp. of Bedford, Massachusets at a rate of about 0.1 to 50 mg per capsule, 1 to 8 capsules being administered daily for an average human. Each capsule to be used in the turbo-inhaler contains the required amount of a Compound of the invention with the remainder of the 20 mg capsule being a pharmaceutically acceptable carrier such as lactose. In a liquid aerosol, a Compound of the invention may be administered using a nebulizer such as, for example, a 'Retec' (trademark) nebulizer, in which the solution is nebulized with compressed air. The aerosol may be administered, for example, at the rate of one to about eight times per day as follows: A nebulizer is filled with a solution of a Compound, for example 3.5 mL of solution containing 10 mg/mL; the solution in the nebulizer is nebulized with compressed air; and the patient breathes normally (tidal volume) for eight minutes with the nebulizer in his mouth.

Alternatively, the mode of adminstration may be oral or parenteral, including subcutaneous deposit by means of an osmotic pump. A compound of the invention may be conventionally formulated in an oral or parenteral dosage form by compounding about 10 to 250 mg per unit of dosage with conventional vehicle, excipient, binder, preservative, stabilizer, flavor or the like as called for by accepted pharmaceutical practice, e.g. as described in U.S. Patent 3,755,340. For parenteral administration, a 1 to 10 mL intravenous, intramuscular or subcutaneous injection would be given containing about 0.02 mg to 10 mg/kg of body weight of a compound of the invention 3 or 4 times daily. The injection would contain a compound of the invention in an aqueous isotonic sterile solution or suspension optionally with a preservative such as phenol or a solubilizing agent such as ethylenediaminetetraacetic acid (EDTA).

For parenteral administration or use in an aerosol, an 10 mg/mL aqueous formulation of an acidic Compound may be prepared, for example by dissolving the Compound (10 mg), dibasic sodium phosphate heptahydrate, USP (11.97 mg), monobasic sodium phosphate, USP (0.74 mg), sodium chloride, USP (4.50 mg) and sufficient 1 N sodium hydroxide solution or 0.05 M monobasic sodium phosphate solution to achieve pH 7.0-7.5 in sufficient water for injection, USP to afford 1.0 mL (1.01 g), followed by aseptic filtration, and sterile storage using standard procedures.

In general, a Compound of the invention will be administered to humans at a daily dose in the range of, for example, 5 to 100 mg of the Compound by aerosol or 50 to 1000 mg intravenously, or a combination of the two. However, it readily will be understood that it may be necessary to vary the dose of the Compound adminstered in accordance with well known medical practice to take account of the nature and severity of the disease under treatment, concurrent therapy, and the age, weight and sex of the patient receiving treatment. It similarly will be understood that generally equivalent amounts of a pharmaceutically acceptable salt of the Compound also may be used. Protocols for the administration of an HLE inhibitor and evaluation of the patients are described in the European Patent Applications with Publication Numbers 458535, 458536, 458537, and 463811 for the treatment or prevention of cystic fibrosis, ARDS, bronchitis, and hemorrhage associated with acute non-lymphocytic leukemia or its therapy, respectively; and a Compound of the invention may be used similarly for the treatment of those diseases and conditions either alone or in combination with another therapeutic agent customarily indicated for the treatment of the particular condition. For therapeutic or prophylactic treatment of a vascular disease or related condition in a mammal in which neutrophils are involved or implicated, a Compound of the invention may conveniently be administered by a parenteral route, either alone or simultaneously or sequentially with other therapeutically active agents customarily administered for the condition.

The invention will now be illustrated by the following non-limiting examples in which, unless stated otherwise:
(i) temperatures are given in degrees Celsius (°C); operations were carried out at room or ambient temperature, that is, at a temperature in the range of 18-25 °C;
(ii) organic solutions were dried over anhydrous sodium sulfate; evaporation of solvent was carried out using a rotary evaporator under reduced pressure (600-4000 pascals; 4.5-30 mm Hg) with a bath temperature of up to 60 °C;
(iii) chromatography means 'flash chromatography' (method of Still) carried out on Merck Kieselgel (Art 9385 from E. Merck, Darmstadt, Germany); if "acidic silica gel" is indicated, material custom prepared by J. T. Baker Chemical Co., Phillipsburg, NJ, USA, and having a pH of about 6 when slurried in water was used; reversed phase chromatography means flash chromatography over octadecylsilane (ODS) coated support having a particle diameter of 32-74 µ, know as "PREP-40-ODS" (Art 731740-100 from Bodman Chemicals, Aston, PA, USA); thin layer chromatography (TLC) was carried out on 0.25 mm silica gel GHLF plates (Art 21521 from Analtech, Newark, DE, USA); reversed phase-TLC (RP-TLC) was carried out Vhatman MKC₁₈F plates (Art 4803-110 from Bodman Chemicals);
(iv) in general, the course of reactions was followed by TLC and reaction times are given for illustration only;
(v) melting points are uncorrected and (dec) indicates decomposition; the melting points given are those obtained for the materials prepared as described; polymorphism may result in isolation of materials with different melting points in some preparations;
(vi) final products had satisfactory nuclear magnetic resonance (NMR) spectra;
(vii) yields are given for illustration only and are not necessarily those which may be obtained by diligent process development; preparations were repeated if more material was required;
(viii) when given, NMR data is in the form of delta values for major diagnostic protons, given in parts per million (ppm) relative to tetramethylsilane (TMS) as an internal standard, determined at 250 MHz using DMSO-d₆ (or DMSO-d₆/D₂O, indicated herein as DMSO/D₂O) as solvent; conventional abbreviations for signal shape are used; for AB spectra the directly observed shifts are reported;
(ix) chemical symbols have their usual meanings; SI units and symbols are used;
(x) reduced pressures are given as absolute pressures in pascals (Pa); elevated pressures are given as gauge pressures in bars; (xi) solvent ratios are given in volume:volume (v/v) terms; and
(xii) mass spectra (MS) were run with an electron energy of 70 electron volts in the chemical ionizaton mode using a direct exposure probe; where indicated ionization was effected by electron impact (EI) or fast atom bombardment (FAB); generally, only peaks which indicate the parent mass are reported.

### EXAMPLES 1-6

The following compounds of Formula I wherein R⁰ is isopropyl, R⁵ is hydrogen, R⁶ is phenyl and R is the indicated value were prepared by oxidation of the corresponding alcohols of formula II using a procedure similar to that which follows:

To a solution of an alcohol of Formula II wherein R⁰ is isopropyl, R⁵ is hydrogen, R⁶ is phenyl and R is the indicated value (0.2 millimolar in dimethylsulfoxide:toluene, 1:1) is added 1-(3-dimethylaminopropyl)-3-ethylcarbodiimide hydrochloride (10 equivalents) and dichloroacetic acid (5 equivalents). After overnight stirring, the reaction mixture is diluted with ethyl acetate; washed (10% hydrochloric acid, saturated aqueous sodium bicarbonate, brine), dried and evaporated to give material which is purified using chromatography, eluting with the indicated solvent, except as otherwise noted, to provide the product.

Example 1: R=benzylsulfonyl: Chromatography solvent: dichloromethane:ethyl acetate (95:5), and drying overnight in a vacuum oven; mp 192-193 °C; TLC: R_{f}=0.29, methanol:dichloromethane (5:95); NMR: 0.85 (d,3), 0.90 (d,3), 2.14 (m,1), 4.57 (m,5), 6.10 (d,1), 7.24 (d,1), 7.36 (m,10), 8.74 (d,1), 8.82 (s,1); MS: m/z=550(M+1).

| Analysis for C₂₆H₂₆F₃N₃O₅: | | | |
|---|---|---|---|
| Calculated: | C, 56.82; | H, 4.77; | N, 7.65 |
| Found: | C, 56.46; | H, 5.03; | N, 7.50 |

Example 2: R=2-(2-pyridyl)ethylsulfonyl: Chromatography solvent: methanol:dichloromethane (2:98), followed by trituration with methyl tert-butyl ether; mp 80-86 °C; TLC: R_{f}=0.43, methanol:dichloromethane (5:95); NMR: 0.76 (d,3), 0.84 (d,3), 2.21 (m,1), 3.24 (t,2), 3.83 (t,2), 4.44 (d,1), 4.58 (d,1), 6.25 (d,1), 7.46 (m,7), 7.74 (dd,1), 8.48 (d,1), 8.73 (d,1), 9.13 (s,1); MS: m/z=565(M+1).

| Analysis for C₂₆H₂₇N₄O₅S·0.3 methyl tert-butyl ether: | | | |
|---|---|---|---|
| Calculated: | C, 54.88; | H, 5.33; | N, 9.31 |
| Found: | C, 54.78; | H, 5.41; | N, 8.94 |

Example 3: R=8-quinolylsulfonyl: Chromatography solvent: dichloromethane:tetrahydrofuran (20:1); TLC: R_{f}=0.39, dichloromethane:methanol (5:1); MS: m/z=587(M+1).

| Analysis for C₂₈H₂₅F₃N₄O₅S·1.0 H₂O: | | | |
|---|---|---|---|
| Calculated: | C, 55.62; | H, 4.50; | N, 9.43 |
| Found: | C, 55.97; | H, 4.46; | N, 9.15 |

Example 4: R=butylsulfonyl: Chromatography solvent: dichloromethane:methanol (95:5); TLC: R_{f}=0.45, dichloromethane:methanol (20:1); MS: m/z=516(M+1).

| Analysis for C₂₃H₂₈F₃N₃O₅S·0.25 H₂O: | | | |
|---|---|---|---|
| Calculated: | C, 53.12; | H, 5.52; | N, 8.08 |
| Found: | C, 53.17; | H, 5.64; | N, 8.01 |

Example 5: R=4-nitrobenzylsulfonyl: Chromatography solvent: dichloromethane:methanol (gradient, 100:0, 99:1, 98:2); TLC: R_{f}=0.45, dichloromethane:methanol (95:5); MS: m/z=595 (M+1).

| Analysis for C₂₆H₂₅F₃N₄O₇S: | | | |
|---|---|---|---|
| Calculated: | C, 52.52; | H, 4.24; | N, 9.42 |
| Found: | C, 52.17; | H, 4.27; | N, 9.49 |

Example 6: R=3-pyridylsulfonyl: Purified by chromatography, with methanol:dichloromethane (2:98) as the eluent for three columns and ethyl acetate:hexane (3:1) as the eluent for a fourth column; TLC: R_{f}=0.35, ethyl acetate:hexane (3:1); MS: m/z=537(M+1).

| Analysis for C₂₄H₂₃F₃N₄O₅S·1.5 H₂O: | | | |
|---|---|---|---|
| Calculated: | C, 51.15; | H, 4.65; | N, 9.94 |
| Found: | C, 51.18; | H, 4.59; | N, 9.37 |

The intermediate alcohols of Formula II used in Examples 1-6 were prepared as follows.

### EXAMPLES 1.a.-6.a.

tert-Butyldimethylsilyl ethers of the alcohols of Formula II wherein R⁰ is isopropyl, R⁵ is hydrogen, R⁶ is phenyl and R is the indicated value were prepared using the following general procedure:

2-(3-Amino-2-oxo-6-phenyl-1,2-dihydro-1-pyridyl)-N-(2-tert-butyldimethylsilyloxy-3,3,3-trifluoro-1-isopropylpropyl)acetamide is sulfonylated, using triethylamine and the required sulfonyl chloride in tetrahydrofuran. The solution is stirred overnight, diluted with ethyl acetate, washed (10 % hydrochloric acid, saturated aqueous sodium bicarbonate, brine), dried and evaporated to give the sulfonamide, which is purified using chromatography, eluting with the indicated solvent, except as otherwise noted.

Example 1.a.: R=benzylsulfonyl: The product was used without additional purification; TLC: R_{f}=0.82, toluene:ethyl acetate (2:1); MS: m/z=666(M+1).

Example 2.a.: R=2-(2-pyridyl)ethylsulfonyl: The product was used without additional purification; TLC: R_{f}=0.42, toluene:ethyl acetate (1:1); MS: m/z=681(M+1).

Example 3.a.: R= 8-quinolylsulfonyl: Chromatography solvent: dichloromethane:methanol (25:1); TLC: R_{f}=0.51, dichloromethane:methanol (20:1); MS: m/z=731(M+1).

Example 4.a.: R=butylsulfonyl; The product was used without additional purification; TLC: R_{f}=0.68, toluene:ethyl acetate (2:1); MS: m/z=632(M+1).

Example 5.a.: R=4-nitrobenzylsulfonyl: Pyridine was used in place of triethylamine and the product was used without additional purification; TLC: R_{f}=0.63, dichloromethane:ethyl acetate (70:30); MS: m/Z=711(M+1).

Example 6.a.: R=3-pyridylsulfonyl: Using dichloromethane in place of tetrahydrofuran and using the hydrochloride of 3-pyridylsulfonyl chloride, with two equivalents of base employed. The resulting material was a mixture of mono- and bis-sulfonylation products. The mixture was used directly in Example 6.b.

The required sulfonyl chloride for Example 6.a. was prepared by a method similar to that described by T.F. Mich, in U.S. Patent 4,315,014, for the preparation of 2-(4-pyridyl)ethylsulfonyl chloride hydrochloride: Purified by trituration with carbon tetrachloride, acetonitrile, and diethyl ether.

### EXAMPLES 1.b.-6.b.

The alcohols of Formula II wherein R⁰ is isopropyl, R⁵ is hydrogen, R⁶ is phenyl and R is the indicated value were prepared using the following general procedure (acetic acid buffered fluoride):

The corresponding tert-butyldimethylsilyl ether prepared in Examples 1.a.-6.a. is dissolved in tetrahydrofuran and treated with tetrabutylammonium fluoride (1 M in tetrahydrofuran, 1.1 equivalents) and glacial acetic acid (1 equivalent). The mixture is stirred for 4.5 hours, diluted with ethyl acetate, washed (water, brine), dried and evaporated to give the product, which is used without further purification, except as otherwise noted.

Example 1.b.: R=benzylsulfonyl: TLC: R_{f}=0.31, toluene:ethyl acetate (2:1); MS: m/z=552(M+1).

Example 2.b.: R=2-(2-pyridyl)ethylsulfonyl: Chromatographed, eluting with methanol:dichloromethane (3:97), followed by trituration with hexane; TLC: R_{f}=0.33, methanol:dichloromethane (5:95); MS: m/z=567(M+1).

Example 3.b.: R=8-quinolylsulfonyl: TLC: R_{f}=0.16, dichloromethane:methanol (20:1); MS: m/z=589(M+1).

Example 4.b.: R=butylsulfonyl: Chromatographed, eluting with dichloromethane:methanol (98:2); TLC: R_{f}=0.5, dichloromethane:- methanol (95:5); MS: m/z=518(M+1).

Example 5.b.: R=4-nitrobenzylsulfonyl: TLC: R_{f}=0.51, dichloromethane:-ethyl acetate (70:30); MS: m/z=597(M+1).

Example 6.b.: R=3-pyridylsulfonyl: The product was a a mixture of mono- and bis-sulfonyl compounds. The mixture was dissolved in tetrahydrofuran and treated with 1 N NaOH at 60 °C for 1.5 hours. Ethyl acetate was added followed by 1 N hydrochloric acid to pH 2. The mixture was diluted with water and the organic layer was dried (MgSO₄), evaporated and purified by chromatography, with methanol:dichloromethane (3:97) as the eluent, to give the mono-sulfonyl compound; TLC: R_{f}=0.40, methanol:dichloromethane (10:90); MS: m/z=539(M+1).

The intermediate 2-(3-amino-2-oxo-6-phenyl-1,2-dihydro-1-pyridyl)-N-(2-tert-butyldimethylsilyloxy-3,3,3-trifluoro-1-isopropylpropyl)acetamide used in Examples 1.a.-6.a. was prepared as described at Example 22.a.-22.e. of European Patent Application, Publication Number 509769; see also preparations described at Example 1.a.-1.h. and Example 14.a. of that application.

### EXAMPLE 7

### 2-(3-Methoxycarbonylmethylsulfonylamino-2-oxo-6-phenyl-1,2-dihydro-1-pyridyl)-N-(3,3,3-trifluoro-1-isopropyl-2-oxopropyl)acetamide.

To a solution of 2-(3-amino-2-oxo-6-phenyl-1,2-dihydro-1-pyridyl)-N-(3,3,3-trifluoro-1-isopropyl-2-oxopropyl)acetamide (0.50 g) in tetrahydrofuran (6 mL), cooled to 0 °C, was added, dropwise, methyl chlorosulfonylacetate (0.29 g). Immediately, triethylamine (0.38 g) was added dropwise to the reaction mixture, generating a color change from light orange to green. After 10 min stirring, the reaction mixture was diluted with 25 mL ethyl acetate and acidified with 1 N aqueous hydrochloric acid. The organic phase was washed (water, brine), dried (magnesium sulfate) and evaporated to yield 0.60 g of an orange foam. Chromatography, using acidic silica gel and eluant of methylene chloride:tetrahydrofuran (20:1), followed by overnight vacuum-drying (40 °C, 27 Pa), yielded a light-yellow foam (0.35 g); mp 165-168 °C; TLC: R_{f}=0.33, dichloromethane:tetrahydrofuran (9:1, trace acetic acid); NMR: 0.90 (2d,6), 2.20 (m,1), 3.70 (s,3), 4.4-4.6 (s and m, 4), 4.65 (t,1), 6.20 (d,1), 7.45 (m,6), 8.75 (d,1), 9.35 (s,1); MS: m/z=532(M+1).

| Analysis for C₂₂H₂₄F₃N₃O₇S: | | | |
|---|---|---|---|
| Calculated: | C, 49.72; | H, 4.55; | N, 7.91 |
| Found: | C, 50.55; | H, 4.79; | N, 7.55 |

The intermediate 2-(3-amino-2-oxo-6-phenyl-1,2-dihydro-1-pyridyl)-N-(3,3,3-trifluoro-1-isopropyl-2-oxopropyl)acetamide may be prepared as dexcribed in European Patent Application, Publication Number 509769 at Example 49 (and the subparts thereunder); see also Example 22.a.-22.b. and Example 167 of that application.

### EXAMPLES 8-12

Using a procedure similar to that described in Example 7, the following compounds of Formula I wherein R⁰ is isopropyl, R⁵ is hydrogen, R⁶ is phenyl and R has the indicated value were prepared by sulfonylation of 2-(3-amino-2-oxo-6-phenyl-1,2-dihydro-1-pyridyl)-N-(3,3,3-trifluoro-1-isopropyl-2-oxopropyl)acetamide with the corresponding sulfonyl chloride. Except as otherwise noted, the product was purified by chromatography over silica gel.

Example 8: R=methylsulfonyl: Chromatography on acidic silica gel, with dichloromethane:tetrahydrofuran (20:1) as the eluent; TLC: R_{f}=0.37, dichloromethane:tetrahydrofuran (9:1, with 2.5% acetic acid); 300 MHz NMR: 0.90 (2d,6), 2.15 (m,1), 3.10 (s,3), 4.50 (q,2), 4.63 (t,1), 6.20 (d,1), 7.40 (m,6), 8.75 (d,1), 8.95 (s,1); MS: m/z=471(M+1).

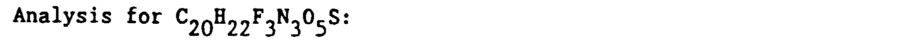

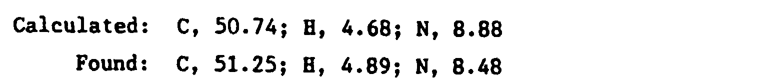

Example 9: R=methylsulfonylmethylsulfonyl: Purified by trituration with ethyl acetate; TLC: R_{f}=0.2, methanol:dichloromethane (5:95); 300 MHz NMR: 0.84 (d,3), 0.89 (d,3), 2.15 (m,1), 3.22 (s,3), 4.48 (q,2), 4.64 (t,1), 5.36 (s,2), 6.23 (d,1), 7.41 (m,6), 8.75 (d,1), 9.71 (s,1); MS: m/z=552(M+1).

| Analysis for C₂₁H₂₄F₃N₃O₇S₂: | | | |
|---|---|---|---|
| Calculated: | C, 45.73; | H, 4.38; | N, 7.62 |
| Found: | C, 45.41; | H, 4.40; | N, 7.59 |

Example 10: R=aminosulfonyl: Recrystallized from hexane:ethyl acetate (1:10); TLC : R_{f}=0.22, dichloromethane:methanol (20:1); MS: m/z=475(M+1).

| Analysis for C₁₉H₂₁F₃N₄O₅S·0.25 H₂O: | | | |
|---|---|---|---|
| Calculated: | C, 47.65; | H, 4.52; | N, 11.70 |
| Found: | C, 47.73; | H, 4.46; | N, 11.60 |

Example 11: R=benzylaminosulfonyl: Purified by trituration with diethyl ether; TLC: R_{f}=0.26, dichloromethane:methanol (20:1); MS: m/z=565(M+1).

| Analysis for C₂₆H₂₇F₃N₄O₅S·0.25 H₂O: | | | |
|---|---|---|---|
| Calculated: | C, 55.31; | H, 4.82; | N, 9.92 |
| Found: | C, 54.91; | H, 4.88; | N, 9.74 |

Example 12: R=trifluoromethylsulfonyl: Chromatography solvent: dichloromethane:methanol (40:1); TLC: R_{f}=0.17, dichloromethane:methanol (20:1); MS: m/z=528(M+1).

| Analysis for C₂₀H₁₉F₆N₃O₅S: | | | |
|---|---|---|---|
| Calculated: | C, 45.54; | H, 3.63; | N, 7.97 |
| Found: | C, 45.49; | H, 3.68; | N,7.76 |

### EXAMPLES 13-25

Using a procedure similar to that described in Example 7, except replacing triethylamine with pyridine, the following compounds of Formula I wherein R⁰ is isopropyl, R⁵ is hydrogen, R⁶ is phenyl and R has the indicated value were prepared by sulfonylation of 2-(3-amino-2-oxo-6-phenyl-1,2-dihydro-1-pyridyl)-N-(3,3,3-trifluoro-1-isopropyl-2-oxopropyl)acetamide with the corresponding sulfonyl chloride and purified by chromatography over silica gel, except as otherwise indicated. If the sulfonyl chloride was a pyridine hydrochloride, an extra equivalent of base was used.

Example 13: R=phenylsulfonyl: Chromatography solvent: dichloromethane:tetrahydrofuran (20:1); TLC: R_{f}=0.31, dichloromethane:methanol (20:1); MS: m/z=536(M+1).

| Analysis for C₂₅H₂₄F₃N₃O₅S: | | | |
|---|---|---|---|
| Calculated: | C, 56.07; | H, 4.52; | N, 7.85 |
| Found: | C, 55.82; | H, 4.66; | N, 7.58 |

Example 14: R=4=chlorophenylsulfonyl: Chromatography solvent: dichloromethane:tetrahydrofuran (20:1); TLC: R_{f}=0.30, dichloromethane:methanol (20:1); MS: m/z=570(M+1).

| Analysis for C₂₅H₂₃ClF₃N₃O₅S: | | | |
|---|---|---|---|
| Calculated: | C, 52.68; | H, 4.07; | N, 7.37 |
| Found: | C, 52.71; | H, 4.25; | N, 7.12 |

Example 15: R=4-methoxyphenylsulfonyl: Chromatography solvent: dichloromethane:tetrahydrofuran (7:1); TLC: R_{f}=0.18, dichloromethane:tetrahydrofuran (5:1); MS: m/z=566 (M+1).

| Analysis for C₂₆H₂₆F₃N₃O₆S·0.25 H₂O: | | | |
|---|---|---|---|
| Calculated: | C, 54.78; | H, 4.69; | N, 7.37 |
| Found: | C, 54.80; | H, 4.69; | N, 7.39 |

Example 16: R=anilinosulfonyl: Chromatography solvent: dichloromethane:acetonitrile (80:20); TLC: R_{f}=0.42, dichloromethane:ethyl acetate (70:30); MS: m/z=551(M+1).

| Analysis for C₂₅H₂₅F₃N₄O₅S·0.5 H₂O: | | | |
|---|---|---|---|
| Calculated: | C, 53.66; | H, 4.68; | N, 10.01 |
| Found: | C, 53.76; | H, 4.68; | N, 9.97 |

Example 17: R=butylaminosulfonyl: Purified by trituration with methyl tert-butyl ether; TLC: R_{f}=0.57, dichloromethane:ethyl acetate (70:30); MS: m/z=531(M+1).

| Analysis for C₂₃H₂₉F₃N₄O₅S: | | | |
|---|---|---|---|
| Calculated: | C, 52.07; | H, 5.51; | N, 10.56 |
| Found: | C, 51.92; | H, 5.52; | N, 10.51 |

Example 18: R=methylaminosulfonyl: Purified by trituration with methyl tert-butyl ether; TLC: R_{f}=S.29, dichloromethane:ethyl acetate (70:30); MS: m/z-489(M+1).

| Analysis for C₂₀H₂₃F₃N₄O₅S: | | | |
|---|---|---|---|
| Calculated: | C, 49.18; | H, 4.74; | N, 11.47 |
| Found: | C, 49.11; | H, 4.75; | N, 11.42 |

Example 19: R=cyclohexylaminosulfonyl: Purified by trituration with methyl tert-butyl ether; TLC: R_{f}=0.58, dichloromethane:ethyl acetate (70:30); MS: m/z=557(M+1).

| Analysis for C₂₅H₃₁F₃N₄O₅S: | | | |
|---|---|---|---|
| Calculated: | C, 53.95; | H, 5.61; | N, 10.06 |
| Found: | C, 53.57; | H, 5.59; | N, 9.97 |

Example 20: R=4-nitrophenylsulfonyl: Chromatography solvent: dichloromethane:methanol (20:1); TLC: R_{f}=0.25, dichloromethane:tetrahydrofuran:acetic acid (100:10:1); MS: m/z=581(M+1).

| Analysis for C₂₅H₂₃F₃N₄O₇S·0.25 H₂O: | | | |
|---|---|---|---|
| Calculated: | C, 51.33; | H, 4.05; | N, 9.58 |
| Found: | C, 51.20; | H, 3.72; | N, 9.41 |

Example 21: R=4-acetylaminophenylsulfonyl: Chromatography solvent: dichloromethane:methanol (20:1); TLC: R_{f}=0.12, dichloromethane:methanol (20:1); MS: m/z=593(M+1).

| Analysis for C₂₇H₂₇F₃N₄O₆S·1.0 H₂O: | | | |
|---|---|---|---|
| Calculated: | C, 53.11; | H, 4.80; | N, 9.18 |
| Found: | C, 53.31; | H, 4.83; | N, 8.94 |

Example 22: R=4-pyridylmethylsulfonyl: Chromatography solvent: dichloromethane:methanol (99:1 to 98:2); TLC: R_{f}=0.26, dichloromethane:methanol (95:5); MS: m/z=551(M+1).

| Analysis for C₂₅H₂₅F₃N₄O₅S·0.5 H₂O: | | | |
|---|---|---|---|
| Calculated: | C, 53.66; | H, 4.68; | N, 10.01 |
| Found: | C, 53.70; | H, 4.60; | N, 9.87 |

Example 23: R=3-pyridylmethylsulfonyl: Chromatography solvent: dichloromethane:methanol (99:1); TLC: R_{f}=0.28, dichloromethane:methanol (95:5); MS: m/z=551(M+1).

| Analysis for C₂₅H₂₅F₃N₄O₅S: | | | |
|---|---|---|---|
| Calculated: | C, 53.66; | H, 4.68; | N, 10.01 |
| Found: | C, 53.75; | H, 4.48; | N, 9.98 |

Example 24: R=tert-butylaminosulfonyl: Chromatography solvent: dichloromethane:ethyl acetate (70:30); TLC: R_{f}=0.55, dichloromethane:ethyl acetate (70:30), MS: m/z= 531(M+1).

| Analysis for C₂₃H₂₉F₃N₄O₅S: | | | |
|---|---|---|---|
| Calculated: | C, 52.07; | H, 5.51; | N, 10.56 |
| Found: | C, 52.19; | H, 5.51; | N, 10.52 |

Example 25: R=4-carboxyphenylsulfonyl: Reverse phase chromatography solvent: methanol:water (50:50); RP-TLC: R_{f}=0.36, methanol:water (65:35); MS: m/z=580(M+1).

| Analysis for C₂₆H₂₄F₃N₃O₇S: | | | |
|---|---|---|---|
| Calculated: | C, 53.88; | H, 4.17; | N, 7.25 |
| Found: | C, 53.95; | H, 4.19; | N, 7.17 |

### EXAMPLE 26

### 2-(2-Oxo-6-phenyl-3-sulfoamino-1,2-dihydro-1-pyridyl)-N-(3,3,3-trifluoro-1-isopropyl-2-oxopropyl)acetamide sodium salt.

Using a procedure similar to that described in Example 7 except that the sulfonyl chloride and triethylamine were replaced by a preformed complex of sulfur trioxide/triethylamine, the title compound was prepared. Purification was performed by ion-exchange chromatography on DOWEX 50, sodium form, with methanol:water (1:10) as the eluant. The appropriate fractions were combined, the methanol and triethylamine evaporated and the remaining solution lyophilized. The residual solid was partially dissolved in warm ethyl acetate, the solid was filtered and the solution evaporated to give a white solid; mp 140 °C (dec); RP-TLC: R_{f}=0.67, methanol:water (65:35); MS: m/z=474(M-1 for free acid), m/z=496(M-1 for sodium salt) by FAB.

| Analysis for C₁₉H₁₉F₃N₃O₆SNa·1.0 H₂O: | | | |
|---|---|---|---|
| Calculated: | C, 44.27; | N, 4.10; | N, 8.15 |
| Found: | C, 44.01; | N, 4.15; | N, 7.84 |

### EXAMPLE 27

### 2-(3-Carboxymethylamino-2-oxo-6-phenyl-1,2-dihydro-1-pyridyl)-N-(3,3,3-trifluoro-1-isopropyl-2-oxopropyl)acetamide.

2-(3-Hethoxycarbonylmethylsulfonylamino-2-oxo-6-phenyl-1,2-dihydro-1-pyridyl)-N-(3,3,3-trifluoro-1-isopropyl-2-oxopropyl)acetamide was dissolved in methanol and treated with 1 N sodium hydroxide. The mixture was diluted with water, acidified with 1 N hydrochloric acid and extracted with ethyl acetate. The organic layers were washed (brine), dried and evaporated to give a solid, which was purified by reverse phase chromatography, with methanol:water (1:1) as the eluent. The resulting material was triturated with hexane to give the title compound; RP-TLC: R_{f}=0.58, methanol:water (65:35); 300 MHz NMR: 0.85 (2d,6), 2.15 (m,1), 4.30 (s,2), 4.50 (q,2), 4.65 (t,1), 6.25 (d,1), 7.45 (m,6), 7.75 (d,1), 9.20 (broad s, 1); MS: m/z=518(M+1).

| Analysis for C₂₁H₂₂F₃N₃O₇S: | | | |
|---|---|---|---|
| Calculated: | C, 48.74; | H, 4.28; | N, 8.12 |
| Found: | C, 48.79; | H, 4.52; | N, 7.85 |

### EXAMPLE 28

### 2-[3-(4-Aminophenylsulfonylamino)-2-oxo-6-phenyl-1,2-dihydro-1-pyridyl]-N-(3,3,3-trifluoro-1-isopropyl-2-oxopropyl)acetamide.

2-[3-(4-Nitrophenylsulfonyl)-2-oxo-6-phenyl-1,2-dihydro-1-pyridyl]-N-(3,3,3-trifluoro-1-isopropyl-2-oxopropyl)acetamide (167 mg) and 10% (w/w) palladium on carbon (42 mg) in a mixture of absolute ethanol (3 mL) and N,N-dimethylformamide (0.5 mL) was shaken under hydrogen (3.5 bar). After 24 hours and 28 hours, 32 mg and 42 mg respectively of 10% (w/w) palladium on carbon, were added. After 32 hours the reaction mixture was filtered through diatomatious earth and the solvent evaporated. The residue was dissolved in tetrahydrofuran, filtered through diatomaceous earth and the solvent evaporated. This residue was dissolved in dichloromethane and the product was precipitated by the addition of hexane to yield the title compound (105 mg) as a hemi-hydrate; mp 140-142 °C (dec); TLC: R_{f}=0.41; dichloromethane:methanol (95:5); MS: m/z=563(M-1) by FAB.

| Analysis for C₂₆H₂₇F₃N₄O₅S·0.5 H₂O: | | | |
|---|---|---|---|
| Calculated: | C, 54.44; | H, 4.92; | N, 9.77 |
| Found: | C, 54.65; | H, 4.88; | N, 9.79 |

### EXAMPLE 29

### 2-[3-(4-Aminobenzylsulfonylamino)-2-oxo-6-phenyl-1,2-dihydro-1-pyridyl]-N-(3,3,3-trifluoro-1-isopropyl-2-oxopropyl)acetamide.

2-[3-(4-Nitrophenylsulfonyl)-2-oxo-6-phenyl-1,2-dihydro-1-pyridyl]-N-(3,3,3-trifluoro-1-isopropyl-2-oxopropyl)acetamide (0.20 g), 10% (w/w) palladium on carbon (0.05 g), and 2.5 mL absolute ethanol were combined and shaken under hydrogen (3.5 bar). After 4 hours the reaction mixture was filtered through diatomaceous earth and evaporated to yield 0.15 g of a light yellow solid. Chromatography, eluting with dichloromethane:methanol (40:1), followed by overnight vacuum drying, (50 °C, 27 Pa), yielded the title compound (0.08 g) as an off-white solid; mp 110-113 °C; TLC: R_{f}=0.19, dichloromethane:methanol (20:1); MS: m/z=551(M+1).

| Analysis for C₂₅H₂₅F₃N₄O₅S·0.75 H₂O: | | | |
|---|---|---|---|
| Calculated: | C, 53.23; | H, 4.74; | N, 9.93 |
| Found: | C, 53.50; | H, 4.68; | N, 9.62 |

### EXAMPLE 30

### 2-[3-(4-Trifluoroacetylaminophenylsulfonylamino)-2-oxo-6-phenyl-1,2-dihydro-1-pyridyl]-N-(3,3,3-trifluoro-1-isopropyl-2-oxopropyl)acetamide.

2-[3-(4-Aminophenylsulfonyl)-2-oxo-6-phenyl-1,2-dihydro-1-pyridyl]-N-(3,3,3-trifluoro-1-isopropyl-2-oxopropyl)acetamide was acylated using a procedure similar to that described in Example 1.a., except substituting trifluoroacetic anhydride for the sulfonyl chloride and dichloromethane for tetrahydrofuran, to give the title compound: Chromatography solvent: dichloromethane:methanol (30:1); TLC: R_{f}=0.33, dichloromethane:tetrahydrofuran:acetic acid (100:10:1); MS: m/z=647(M+1).

| Analysis for C₂₇H₂₄F₆N₄O₆S: | | | |
|---|---|---|---|
| Calculated: | C, 50.16; | H, 3.74; | N, 8.67 |
| Found: | C, 50.42; | H, 3.87; | N,8.46 |

### EXAMPLES 31-41

Using a procedure similar to that described in Example 7, except replacing triethylamine with pyridine, the following compounds of Formula I wherein R⁰ is isopropyl, R⁵ is hydrogen, R⁶ is phenyl and R has the indicated value were prepared by sulfonylation of 2-(3-amino-2-oxo-6-phenyl-1,2-dihydro-1-pyridyl)-N-(3,3,3-trifluoro-1-isopropyl-2-oxopropyl)acetamide using the corresponding sulfonyl chloride and purified by chromatography over silica gel, except as otherwise indicated.

Example 31: R=trifluoromethylsulfonyl: Chromatography solvent: dichloromethane:methanol (40:1); TLC: R_{f}=0.17, dichloromethane:methanol (20:1); NMR: 8.75 (s,1), 7.45 (m,7), 6.22 (d,2), 4.63 (m,1), 4.50 (dd,2), 2.16 (m,1), 0.88 (dd,6); MS: m/z=528(M+1).

| Analysis for C₂₀H₁₉F₆N₃O₅S: | | | |
|---|---|---|---|
| Calculated: | C, 45.54; | H, 3.63; | N, 7.97 |
| Found: | C, 45.64; | H, 3.66; | N, 7.78 |

Example 32: R=3-nitrophenylsulfonyl: Chromatography solvent: dichloromethane:methanol (30:1); TLC: R_{f}=0.5, dichloromethane:methanol:acetic acid (99:10:1); NMR: 10.20 (s,1), 8.65 (m,2), 8.47 (d,1), 8.30 (d,1), 7.85 (dd,1), 7.47 (m,5), 7.30 (d,1), 6.18 (d,1), 4.58 (dd,1), 4.35 (dd,2), 2.14 (m,1), 0.82 (dd,6); MS: m/z=581(M+1).

| Analysis for C₂₅H₂₃N₄O₇F₃S: | | | |
|---|---|---|---|
| Calculated: | C, 51.72; | H, 3.99; | N, 9.65 |
| Found: | C, 51.80; | H, 4.08; | N, 9.57 |

Example 33: R=isopropylaminosulfonyl: Chromatography solvent: dichloromethane:tetrahydrofuran (85:15), followed by trituration with diethyl ether; TLC: R_{f}=0.44, dichloromethane:-methanol (20:1); NHR: 8.73 (d,1), 8.32 (s,1), 7.58 (m ,1), 7.43 (m,6), 6.72 (d,1), 4.63 (dd,1), 4.50 (dd,2), 3.40 (m,1), 2.15 (m,1), 1.05 (m,1), 0.89 (d,3), 0.83 (d,3); MS: m/z=517(M+1).

| Analysis for C₂₂H₂₇F₃N₄O₅S: | | | |
|---|---|---|---|
| Calculated: | C, 51.16; | H, 5.27; | N, 10.85 |
| Found: | C, 50.90; | H, 5.24; | N, 10.77 |

Example 34: R=4-(N,N-dimethylcarbamoylmethyl)phenylsulfonyl: Purified by trituration with diethyl ether; TLC: R_{f}=0.21, dichloromethane:methanol (20:1); NMR (DMSO/D₂O): 7.88 (d,2), 7.42 (m,8), 6.16 (d,1), 4.42 (dd,2), 4.05 (d,1), 3.80 (s,2), 3.03 (s,3), 2.84 (s,3), 2.15 (m,1), 0.85 (dd,6); MS: m/z=621(M+1).

| Analysis for C₂₉H₃₁F₃N₄O₆S·0.50 H₂O: | | | |
|---|---|---|---|
| Calculated: | C, 55.32; | H, 5.12; | N, 8.90 |
| Found: | C, 55.33; | H, 5.13; | N, 8.83 |

Example 35: R=benzoylaminosulfonyl: Purified by trituration with methyl tert-butyl ether; TLC: R_{f}=0.36, dichloromethane:methanol (9:1); NMR (DXSO/D₂O): 6.25 (d,1, J=7.7), 4.55 (d,1, J=16.5), 4.40 (d,1, J=16.5), 4.02 (d,1, J=2.9), 2.22 (m,1), 0.84 (d,3, J=6.8), 0.76 (d,3, J=6.8); MS: FAB m/z=579(M+1), 577(M-1).

| Analysis for C₂₆H₂₅F₃N₄O₆S: | | | |
|---|---|---|---|
| Calculated: | C, 53.98; | H, 4.36; | N, 9.68 |
| Found: | C, 53.65; | H, 4.44; | N, 9.67 |

Example 36: R=methoxycarbonylaminosulfonyl: Purified by sequential trituration, first with diethyl ether:hexanes (1:1) then with methyl tert-butyl ether; TLC: R_{f}=0.20, dichloromethane:methanol (9:1); NMR (DMSO/D₂O): 7.44 (m,6), 6.25 (d,1, J=7.5), 4.56 (d,1, J=16.4), 4.42 (d,1, J=16.4), 4.04 (d,1, J=2.6), 3.65 (s,3), 2.23 (m,1), 0.85 (d,3, J=6.6), 0.78 (d,3, J=6.7); MS: FAB m/z=533(M+1), 531(M-1).

| Analysis for C₂₁H₂₃F₃N₄O₇S·0.5 H₂O: | | | |
|---|---|---|---|
| Calculated: | C, 46.58; | H, 4.47; | N, 10.35 |
| Found: | C, 46.77; | H, 4.39; | N, 10.29 |

Example 37: R=2,2,2-trifluoroethylsulfonyl: Purified by trituration with methyl tert-butyl ether:hexanes; TLC: R_{f}=0.83, dichloromethane:tetrahydrofuran:acetic acid (100:10:1); NMR (DMSO/D₂O): 7.45 (m,6), 6.28 (d,1, J=7.6), 4.52 (m,4), 4.05 (d,1, J=2.6), 2.23 (m,1), 0.86 (d,3, J=6.8), 0.79 (d,3, J=6.8); MS: m/z=542(M+1).

| Analysis for C₂₁H₂₁F₆N₃O₅S·0.5 H₂O: | | | |
|---|---|---|---|
| Calculated: | C, 45.82; | H, 4.03; | N, 7.63 |
| Found: | C, 46.11; | H, 4.02; | N, 7.60 |

Example 38: R=2,2,2-trifluoroethylaminosulfonyl: Purified by trituration with diethyl ether:ethyl acetate (20:1); TLC: R_{f}=0.75, dichloromethane:tetrahydrofuran (9:1); NMR (DMSO/D₂O): 7.44 (m,6), 6.23 (d,1, J=7.6), 4.56 (d,1, J=16.5), 4.31 (d,1, J=16.5), 4.06 (d,1, J=2.9), 3.71 (q,2, J=9.6), 2.24 (m,1), 0.86 (d,3, J=6.8), 0.79 (d,3, J=6.8); MS: m/z=557(M+1).

| Analysis for C₂₁H₂₂F₆N₄O₅S·0.5 H₂O: | | | |
|---|---|---|---|
| Calculated: | C, 44.60; | H, 4.10; | N, 9.91 |
| Found: | C, 44.56, | H, 3.92; | N, 9.98 |

Example 39: R=acetylaminosulfonyl: Reverse phase chromatography solvent: methanol:water (50:50); TLC: R_{f}=0.27, dichloromethane:methanol (90:10); NMR: 7.70 (d,1, J=7.6), 7.43 (m,5), 6.24 (d,1, J=7.6), 4.56 (d,1, J=16.4), 4.41 (d,1, J=16.4), 4.04 (d,1), 2.22 (m,1). 1.96 (s,3), 0.85 (d,3, J=6.8), 0.78 (d,3, J=6.8); MS: FAB m/z=517(M+1), 515(M-1).

| Analysis for C₂₁H₂₃F₃N₄O₆S·1.0 H₂O: | | | |
|---|---|---|---|
| Calculated: | C, 47.19; | H, 4.71; | N, 10.48 |
| Found: | C, 47.13; | H, 4.62; | N, 10.52 |

Example 40: R=ethoxycarbonylaminosulfonyl: Purified by trituration using hexane:diethyl ether (5:1); TLC: R_{f}=0.16, dichloromethane:methanol (10:1); NMR (DMSO/D20): 7.44 (m,6), 6.26 (d,1), 4.48 (dd,2), 4.12 (q,2), 4.05 (d,1), 2.25 (m,1), 1.80 (t,3), 0.85 (d,3), 0.78 (d,3); MS: m/z=FAB 547(M+1), 545(M-1).

| Analysis for C₂₂H₂₅F₃N₄O₇S: | | | |
|---|---|---|---|
| Calculated: | C, 48.35; | H, 4.61; | N, 10.25 |
| Found: | C, 48.16, | H, 4.69; | N, 10.06 |

Example 41: R=cyanomethylsulfonyl: Chromatography solvent: dichloromethane:methanol (9:1), followed by trituration with diethylether; TLC: R_{f}=0.5, dichloromethane:methanol (9:1); NMR (DMSO/D₂O): 7.47 (m,6), 6.24 (d,1, J=7.5), 4.57 (d,1, J=16.3), 4.42 (d,1, J=16.3), 4.06 (d,1, J=2.9), 2.22 (m,1), 0.86 (d,3, J=6.8), 0.78 (d,3, J=6.8); MS: m/z=499(M+1).

| Analysis for C₂₁H₂₁F₃N₄O₅S·0.25 H₂O: | | | |
|---|---|---|---|
| Calculated: | C, 50.15; | H, 4.31; | N, 11.14 |
| Found: | C, 50.14; | H, 4.10; | N, 11.01 |

### EXAMPLE 42

### 2-[3-(3-Aminophenylsulfonylamino)-2-oxo-6-phenyl-1,2-dihydro-1-pyridyl]-N-(3,3,3-trifluoro-1-isopropyl-2-oxopropyl)acetamide.

Using a procedure similar to that described in Example 29, 2-[3-(3-nitrophenylsulfonylamino)-2-oxo-6-phenyl-1,2-dihydro-1-pyridyl]-N-(3,3,3-trifluoro-1-isopropyl-2-oxopropyl)acetamide was converted into the title compound. Chromatography solvent: dichloromethane:methanol (40:1); TLC: R_{f}=0.20, dichloromethane:-methanol (20:1); NMR: 9.43 (s,1), 8.62 (d,1), 7.48 (m,6), 7.18 (dd,1), 7.09 (dd,1), 7.03 (d,1), 6.77 (dd,1), 6.15 (d,1), 5.62 (broad s,2), 4.63 (m,1), 4.45 (dd,2), 2.15 (m,1), 0.87 (dd,6); MS: m/z=551(M+1).

| Analysis for C₂₅H₂₅N₄O₅F₃S: | | | |
|---|---|---|---|
| Calculated: | C, 54.54; | H, 4.58; | N, 10.18 |
| Found: | C, 54.56; | H, 4.74; | N, 10.06 |

### EXAMPLE 43

### 2-[3-(3-Acetylaminophenylsulfonylamino)-2-oxo-6-phenyl-1,2-dihydro-1-pyridyl]-N-(3,3,3-trifluoro-1-isopropyl-2-oxopropyl)acetamide.

Using a procedure similar to that described in Example 7.1., except replacing trifluoroacetic anhydride with acetic anydride, 2-[3-(3-aminophenylsulfonylamino)-2-oxo-6-phenyl-1,2-dihydro-1-pyridyl]-N-(3,3,3-trifluoro-1-isopropyl-2-oxopropyl)acetamide was converted into the title compound. Chromatography solvent: dichloromethane:methanol (35:1); TLC: R_{f}=0.24, dichloromethane:-methanol (20:1); NMR (DMSO/D₂O): 8.22 (s,1) 7.73 (d,1), 7.60 (d,1), 7.5 (br m, 7), 6.16 (d,1), 4.40 (m,2), 4.02 (d,1), 2.12 (m,1), 2.09 (s,3), 0.80 (d,2); MS: m/z=593(M+1).

| Analysis for C₂₇H₂₇F₃N₄O₆S·0.50 H₂O: | | | |
|---|---|---|---|
| Calculated: | C, 53.91; | H, 4.69; | N, 9.31 |
| Found: | C, 54.07; | H, 4.86; | N, 9.10 |

### EXAMPLE 44

### 2-[3-[4-(N-Methylsulfonylcarbamoyl)phenylsulfonylamino]-2-oxo-6-phenyl-1,2-dihydro-1-pyridyl]-N-(3,3,3-trifluoro-1-isopropyl-2-oxopropyl)acetamide.

A solution of 2-[3-(4-carboxyphenylsulfonylamino)-2-oxo-6-phenyl-1,2-dihydro-1-pyridyl]-N-(3,3,3-trifluoro-1-isopropyl-2-oxopropyl)acetamide (0.600 g), methanesulfonamide (0.1983 g), 1-(3-dimethylaminopropyl)-3-ethylcarbodiimide hydrochloride (0.2327 g), and 4-dimethylaminopyridine (0.1472 g) in dichloromethane (4.5 mL) was stirred. After 4 hours and 7 hours, 0.0167 g and 0.0172 g of 1-(3-dimethylaminopropyl)-3-ethylcarbodiimide hydrochloride was added respectively and stirring was continued for 24 hours. Methanesulfonamide (0.0180 g) was added, the solution was stirred for 25 hours. The reaction was diluted with ethyl acetate (100 mL), and was washed (0.1 N hydrochloric acid (twice), water, brine), dried (sodium sulfate) and evaporated to give an orange brown solid. Reverse phase chromatography, eluting with methanol:water (40:60), followed by evaporation of the methanol and additon of 1N hydrochloric acid precipitated the title compound as a white powder (0.196 g). RP-TLC R_{f}=0.66, methanol:water 65:35 pH 6.7; NMR (DMSO/D20): 8.06 (s,4), 7.64 (d,1), 7.41 (m,6), 6.16 (d,1), 4.48 (d,1), 4.32 (d,1), 4.03 (d,1), 3.37 (s,3), 2.21 (m,1), 0.82 (d,3), 0.75 (d,3); MS: m/z=657(M+1).

| Analysis for C₂₇H₂₇F₃N₄O₈S₂·1.0 H₂O: | | | |
|---|---|---|---|
| Calculated: | C, 48.07; | H, 4.33; | N, 8.30 |
| Found: | C, 47.80; | H, 4.29; | N, 8.20 |

### EXAMPLES 45-46

Using a procedure similar to that described in Example 44, except replacing methanesulfonamide with the required sulfonamide, the following compounds of Formula I wherein R⁰ is isopropyl, R⁵ is hydrogen, R⁶ is phenyl and R has the indicated value were prepared.

Example 45: R=4-[N-(4-chlorophenylsulfonyl)carbamoyl]phenylsulfonyl: Chromatography solvent: dichloromethane:methanol (50:1); TLC: R_{f}=0.31, dichloromethane:methanol:acetic acid (100:5:1); NMR: 9.87 (s,1), 8.70 (d,1), 8.01 (d,2), 7.91 (m,4), 7.57 (d,2), 7.38 (m,6), 6.13 (d,1), 4.60 (m,1), 4.38 (dd,2), 2.12 (m,1), 0.85 (dd,6); MS: FAB m/z=751(M-1), 753(M+1).

| Analysis for C₃₂H₂₈ClF₃N₄O₈S₂·1.0 H₂O: | | | |
|---|---|---|---|
| Calculated: | C, 49.84; | H, 3.92; | N, 7.27 |
| Found: | C, 49.63; | H, 3.77; | N, 7.09 |

Example 46: R=4-[N-(2-methylphenylsulfonyl)carbamoyl]phenylsulfonyl: Reverse phase chromatography solvent: methanol:water (40:60); RP-TLC: R_{f}=0.48, methanol:water (65:35 at pH 6.7); NMR: 10.01 (s,1), 8.68 (d,1), 8.02 (m,1), 7.35 (m,1), 6.12 (d,1), 4.59 (dd,1), 4.44 (d,1), 4.33 (d,1), 2.61 (s,3), 2.11 (m,1), 0.85 (d,3). 0.79 (d,3); MS: m/z=733(M+1).

| Analysis for C₃₃H₃₁F₃N₄O₈S₂·0.5H₂O: | | | |
|---|---|---|---|
| Calculated: | C, 53.45; | H, 4.35; | N, 7.55 |
| Found: | C, 53.37; | H, 4.44; | N, 7.73 |

### EXAMPLE 47

### 2-[3-(4-Methylsulfonylaminophenylsulfonylamino)-2-oxo-6-phenyl-1,2-dihydro-1-pyridyl]-N-(3,3,3-trifluoro-1-isopropyl-2-oxopropyl)acetamide.

Using a procedure similar to that described in Example 30, except using methanesulfonyl chloride in place of trifluoroacetic anhydride, the title compound was prepared. Chromatography solvent: dichloromethane:tetrahydrofuran (20:1); TLC: R_{f}=0.23, dichloromethane:tetrahydrofuran:acetic acid (99:10:1); NMR: 10.45 (s,1), 9.60 (s,1), 8.70 (d,1), 7.90 (d,2), 7.37 (d,2), 6.14 (d,1), 4.60 (dd,1), 4.41 (dd,2), 3.15 (s,3), 2.15 (m,1), 0.88 (dd,6); MS: m/z=629(M+1).

| Analysis for C₂₆H₂₇F₃N₄O₇S₂·0.05 C₁₅H₂₄O: | | | |
|---|---|---|---|
| Calculated: | C, 50.22; | H, 4.44; | N, 8.76 |
| Found: | C, 50.20; | H, 4.49; | N, 8.69 |

### EXAMPLE 48

### 2-[3-(4-Methoxycarbonylaminophenylsulfonylamino)-2-oxo-6-phenyl-1,2-dihydro-1-pyridyl]-N-(3,3,3-trifluoro-1-isopyropyl-2-oxopropyl)acetamide.

Using a procedure similar to that described in Example 30, except using methyl chloroformate in place of trifluoroacetic anhydride, and purification by trituration using hexane:diethyl ether (2:1), the title compound was obtained; TLC: R_{f}=0.26, chloroform:tetrahydrofuran:acetic acid (99:10:1); NMR (DMSO/D₂O): 10.12 (s,1), 7.85 (d,2), 7.62 (d,2), 7.40 (m,6), 6.15 (d,1), 4.40 (q,2), 4.03 (d,1), 2.22 (m,1), 0.83 (d,3), 0.76 (d,3); MS: m/z=609(M+1).

| Analysis for C₂₇H₂₇F₃N₄O₇S·0.5 H₂O: | | | |
|---|---|---|---|
| Calculated: | C, 52.51; | H, 4.56; | N, 9.22 |
| Found: | C, 52.63; | H, 4.57; | N, 8.87 |

### EXAMPLES 49-50

Using a procedure similar to that described in Example 7, except using pyridine in place of triethylamine, the following compounds of Formula I wherein R⁰ is isopropyl, R⁵ and R⁶ are each hydrogen and R has the indicated value were prepared by sulfonylation of 2-(3-amino-2-oxo-1,2-dihydro-1-pyridyl)-N-(3,3,3-trifluoro-1-isopropyl-2-oxopropyl)acetamide (Example 75 in European Patent Application, Publication Number 509769) with the corresponding sulfonyl chloride. Except as otherwise noted, the product was purified by chromatography over silica gel.

Example 49: R=4-acetylaminophenylsulfonyl: Chromatography solvent: dichloromethane:methanol (gradient 98:2 to 95:5); TLC: R_{f}=0.46, dichloromethane:methanol (90:10); NMR: 10.31 (s,1), 9.35 (s,1), 8.87 (d,1, J=6.7), 7.78 (d,2), 7.70 (d,2), 7.29 (m,2), 6.17 (t,1, J=7.1), 4.63 (m,3), 2.18 (m,1), 2.06 (s,3), 0.94 (d,3, J=6.8), 0.91 (d,3, J=6.8); MS: m/z=417(M+1).

| Analysis for C₂₁H₂₃F₃N₄O₆S: | | | |
|---|---|---|---|
| Calculated: | C, 48.84; | H, 4.49; | N, 10.85 |
| Found: | C, 48.80; | H, 4.56; | N, 10.50 |

Example 50: R=benzylsulfonyl: Purified by recrystallization from ethyl acetate; TLC: R_{f}=0.41, dichloromethane:methanol (95:5); NMR: 8.95 (d,1, J=6.5), 8.74 (s,1), 7.40 (dd,1), 7.33 (s,5), 7.18 (dd,1, J=1.7, 7.3), 6.17 (t,1, J=7.1), 4.55 (s,2), 2.21 (m,1), 0.97 (d,3, J=6.8), 0.95 (d,3, J=6.8); MS: m/z=474(M+1).

| Anaylsis for: C₂₀H₂₂F₃N₄O₅S: | | | |
|---|---|---|---|
| Calculated: | C, 50.74; | H, 4.68; | N, 8.88 |
| Found: | C, 50.76; | H, 4.67; | N, 8.86 |

### EXAMPLE 51

### 2-(3-Ethylamino-2-oxo-6-phenyl-1,2-dihydro-1-pyridyl)-N-(3,3,3-trifluoro-1-isopropyl-2-oxopropyl)acetamide.

To a solution of 2-(3-amino-2-oxo-6-phenyl-1,2-dihydro-1-pyridyl-N-(3,3,3-trifluoro-1-isopropyl-2-oxopropyl)acetamide (0.305 g) and 2,6-lutidine (0.1 mL) in dimethylformamide (4.5 mL) was added ethyl iodide (0.09 mL). After 20 hours, further charges of 2,6-lutidine (0.1 mL) and ethyl iodide (0.09 mL) were made. After 5 hours, the reaction mixture was added to ethyl acetate and water and the organic phase was separated, washed (brine), dried (MgSO₄), and evaporated. Chromatography (twice), eluting with dichloromethane:methanol (gradient, 99.5:0.5, 99:1) gave the title compound (68 mg) as a yellow solid; TLC: R_{f}=0.42, dichloromethane:methanol (98:2); MS: m/z=424(M+1).

| Analysis for C₂₁H₂₄F₃N₃O₃: | | | |
|---|---|---|---|
| Calculated: | C, 59.56; | H, 5.71; | N, 9.92 |
| Found: | C, 59.17; | H, 5.76; | N, 9.52 |

### EXAMPLE 52

### 2-(2-Oxo-3-phenethylamino-6-phenyl-1,2-dihydro-1-pyridyl)-N-(3,3,3-trifluoro-1-isopropyl-2-oxopropyl)acetamide.

Using a procedure similar to that described for Example 51, but using 2-phenethyl bromide and sodium iodide in place of ethyl iodide, omitting the second addition of reagents and purifying by chromatography, eluting with dichloromethane:methanol (gradient, 99.5:1, 97:3), the title product was obtained as a light yellow foam; TLC: R_{f}=0.41, dichloromethane:methanol (98:2); MS: m/z=500(M+1).

| Analysis for C₂₇H₂₈F₃N₃O₃·0.4 H₂O: | | | |
|---|---|---|---|
| Calculated: | C, 63.99; | H, 5.72; | N, 8.29 |
| Found: | C, 63.93; | H, 5.62; | N, 8.29 |

### EXAMPLE 53

### 2-(3-Methylamino-2-oxo-6-phenyl-1,2-dihydro-1-pyridyl)-N-(3,3,3-trifluoro-1-isopropyl-2-oxopropyl)acetamide.

Using a procedure similar to that described under Example 49.j. of European Patent Application, Publication Number 509769, for the basic hydrolysis of the analogous 3-(N-trifluoroacetylamino)compound to the 3-amino-compound, but using the following extractive work-up, the title compound was prepared from 2-[3-(N-trifluoroacetyl-N-methylamino)-2-oxo-6-phenyl-1,2-dihydro-1-pyridyl]-N-(3,3,3-trifluoro-1-isopropyl-2-oxopropyl)acetamide. On completion of hydrolysis, ethyl acetate and brine were added. The organic phase was separated, washed (brine), dried (HgSO₄), and evaporated. The resultant solid was triturated with diethyl ether, chromatographed, eluting with dichloromethane:methanol (96:4), and dried overnight under vacuum to give the title product as a white solid; TLC: R_{f}=0.20, dichloromethane:methanol (96:4); MS: m/z=410(M+1).

| Analysis for C₂₀H₂₂F₃N₃O₃·0.5 H₂O: | | | |
|---|---|---|---|
| Calculated: | C, 57.41; | H, 5.54; | N, 10.04 |
| Found: | C, 57.42; | H, 5.34; | N, 9.78 |

The starting material was prepared as follows.

### a. 2-[3-(N-Trifluoroacetyl-N-methylamino)-2-oxo-6-phenyl-1,2-dihydro-1-pyridyl)-N-(3,3,3-trifluoro-1-isopropyl-2-oxopropyl)acetamide.

To a solution of 2-(3-trifluoroacetylamino-2-oxo-6-phenyl-1,2-dihydro-1-pyridyl]-N-(3,3,3-trifluoro-1-isopropyl-2-oxopropyl)acetamide (Example 167 in European Patent Application, Publication Number 509769) (200 mg) in dimethylformamide (2 mL) was added sodium carbonate (128 mg) and methyl iodide (130 µL). The mixture was stirred in a stoppered vessel overnight. Ethyl acetate and brine were added. The organic phase was separated, washed (brine), dried (MgSO₄), and evaporated. Chromatography, eluting with dichloromethane:methanol (98:2), gave the title product (80 mg) as a white solid; TLC: R_{f}=0.15, dichloromethane:methanol (98:2); MS: m/z=506(M+1).

### EXAMPLE 54

### 2-[3-(4-Fluorobenzylamino)-2-oxo-6-phenyl-1,2-dihydro-1-pyridyl]-N-(3,3,3-trifluoro-1-isopropyl-2-oxopropyl)acetamide.

2-[3-[N-(4-Fluorobenzyl)-N-trifluoroacetylamino]-2-oxo-6-pheny-1,2-dihydro-1-pyridyl]-N-(3,3,3-trifluoro-1-isopropyl-2-oxopropyl)acetamide was subjected to a procedure similar to that described in Example 53, but purifying by chromatography, eluting with dichloromethane:methanol (gradient, 99.5:0.5, 99:1), to give the title compound; TLC: R_{f}=0.45, dichloromethane:methanol (98:2); MS: m/z=504(M+1).

| Analysis for C₂₆H₂₅F₄N₃O₃·0.3 H₂O: | | | |
|---|---|---|---|
| Calculated: | C,61.36; | H, 5.07; | N, 8.25 |
| Found: | C,61.45; | H, 5.00; | N, 8.23 |

The starting material was prepared as follows.

### a. 2-[3-[N-Trifluoroacetyl-N-(4-fluorobenzyl)amino]-2-oxo-6-phenyl-1,2-dihydro-1-pyridyl]-N-(3,3,3-trifluoro-1-isopropyl-2-oxopropyl)acetamide

Using a procedure similar to that described for Example 53.a. except using 4-fluorobenzyl bromide and sodium iodide in place of methyl iodide and purifying by chromatography, eluting with dichloromethane:methanol (gradient, 99.5:0.5, 98:2), the title product was prepared as a colorless gum; TLC: R_{f}=0.29, dichloromethane:acetone (95:5); MS: m/z=600(M+1).

### EXAMPLE 55

2-[3-[N-Trifluoroacetyl-N-(4-methoxybenzyl)amino]-2-oxo-6-phenyl-1,2-dihydro-1-pyridyl]-N-(3,3,3-trifluoro-1-isopropyl-2-oxopropyl)acetamide was subjected to a procedure similar to that described in Example 53, but purifying by chromatography, eluting with dichloromethane:methanol (gradient, 99:1, 98:2), to give the title compound as a white solid; TLC: R_{f}=0.24, dichloromethane:methanol (98:2); MS: m/z=516(M+1).

| Analysis for C₂₇H₂₈F₃N₃O₄·0.5 H₂O: | | | |
|---|---|---|---|
| Calculated: | C, 61.83; | H, 5.57; | N, 8.01 |
| Found: | C, 61.58; | H, 5.57; | N, 7.77 |

The starting material was prepared as follows.

### a. 2-[3-[N-(4-Methoxybenzyl)-N-trifluoroacetylamino]-2-oxo-6-phenyl-1,2-dihydro-1-pyridyl]-N-(3,3,3-trifluoro-1-isopropyl-2-oxopropyl)acetamide.

Using a procedure similar to that described for Example 53.a., except using 4-methoxybenzyl bromide and sodium iodide in place of methyl iodide and purifying by chromatography, eluting with dichloromethane:methanol (99:1), the title compound was prepared; TLC: R_{f}=0.33, dichloromethane:methanol (98:2); MS: m/z=612(M+1).

### EXAMPLE 56

### 2-(3-Benzylamino-2-oxo-6-phenyl-1,2-dihydro-1-pyridyl)-N-(3,3,3-trifluoro-1-isopropyl-2-oxopropyl)acetamide.

2-[3-(N-Benzyl-N-trifluoroacetylamino)-2-oxo-6-phenyl-1,2-dihydro-1-pyridyl)-N-(3,3,3-trifluoro-1-isopropyl-2-oxopropyl)acetamide was subjected to a procedure similar to that described for Example 53, but purifying by chromatography, eluting with dichloromethane:methanol (gradient, 99.5:0.5, 98:2), to give the title compound as a white solid; TLC: R_{f}=0.45, dichloromethane:methanol (98:2); MS: m/z=486(M+1).

| Analysis for C₂₆H₂₆F₃N₃O₃·0.2 H₂O: | | | |
|---|---|---|---|
| Calculated: | C, 63.84; | H, 5.44; | N, 8.59 |
| Found: | C, 63.86; | H, 5.68; | N, 8.31 |

The starting material was prepared as follows.

### a. 2-[3-(N-Trifluoroacetyl-N-benzylamino)-2-oxo-6-phenyl-1,2-dihydro-1-pyridyl)-N-(3,3,3-trifluoro-1-isopropyl-2-oxopropyl)acetamide.

Using a procedure similar to that described for Example 53.a. except employing benzyl bromide and sodium iodide in place of methyl iodide, heating the reaction mixture at 50 °C, and purifying by chromatography, eluting with dichloromethane:methanol (99:1), the title product was prepared; R_{f}=0.35, dichloromethane:methanol (98:2); MS: m/z-582(M+1).

### EXAMPLE 57

### 2-[3-(2,2,2-Trifluoroethoxycarbonylamino-2-oxo-6-phenyl-1,2-dihydro-1-pyridyl]-N-(3,3,3-trifluoro-1-isopropyl-2-oxopropylpropyl)acetamide.

To a solution of 2-(3-amino-2-oxo-6-phenyl-1,2-dihydro-1-pyridyl)-N-(3,3,3-trifluoro-1-isopropyl-2-oxopropyl)acetamide (400 mg) in dichloromethane (8 mL) at 0 °C was added pyridine (320 mg) followed by 2,2,2-trifluoroethyl chloroformate (180 uL). After 1 hour the reaction mixture was diluted with diethyl ether and quenched with ice. The phases were separated; and the organic phase was washed (dilute hydrochloric acid, brine), dried and evaporated to afford a gummy solid. This solid was triturated with diethyl ether:hexanes (10 mL, 1:1) to afford a white powder which was collected by filtration and dried under vacuum to yield the title compound (388 mg); mp 196-198 °C; TLC: R_{f}=0.68, chloroform:methanol (20:1); NMR (DMSO/D₂O): 7.91 (d,1, J=8.19), 7.30-7.50 (m,5), 6.29 (d,1, J=8.19), 4.83 (d,1, J=9), 4.75 (d,1, J=9), 4.71 (d,1, J=16), 4.47 (d,1, J=16.4), 2.23 (m,1), 0.86 (d,3, J=6.7), 0.80 (d,3, J=6.7); MS: m/z=522(M+1).

| Analysis for C₂₂H₂₁F₆N₃O₅·0·5 H₂O: | | | |
|---|---|---|---|
| Calculated: | C, 49.82; | H, 4.18; | N, 7.92 |
| Found: | C, 49.96; | H, 4.21; | N, 7.80 |

The intermediate 2,2,2-trifluoroethyl chloroformate was prepared using a procedure similar to that described in U.S. Patent Number 3,852,464, except that bis(trichloromethyl) carbonate was used in place of phosgene.

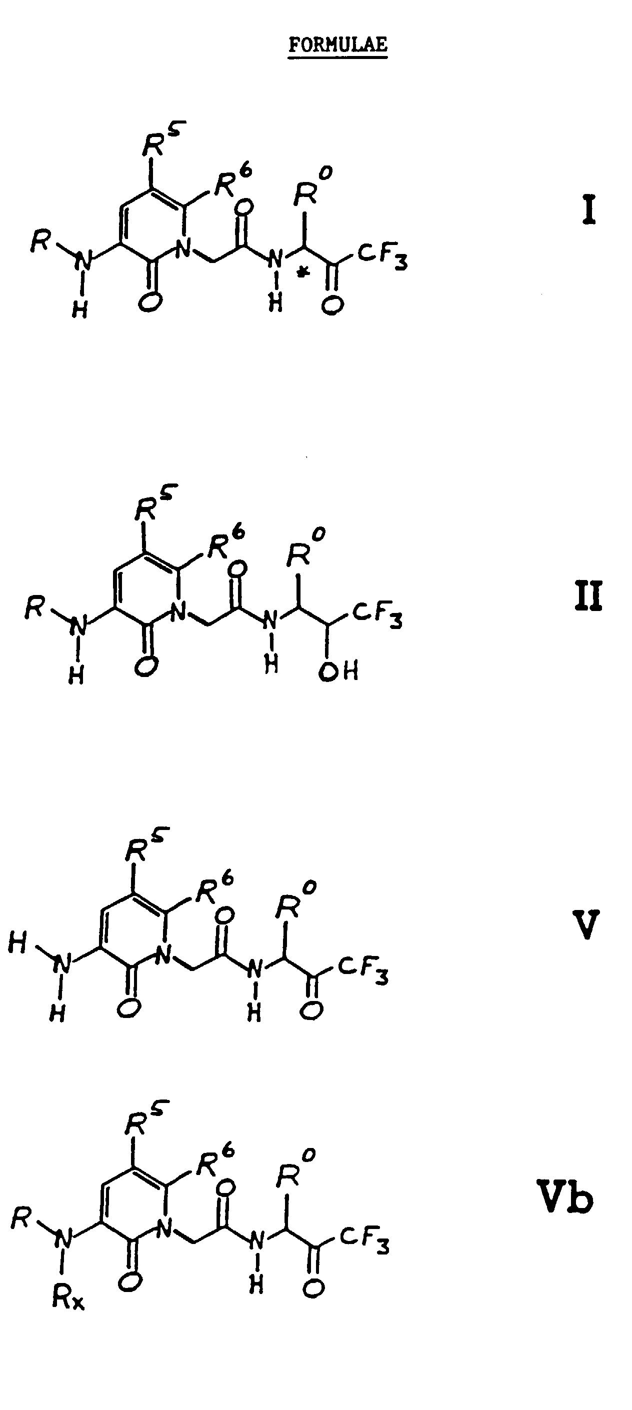

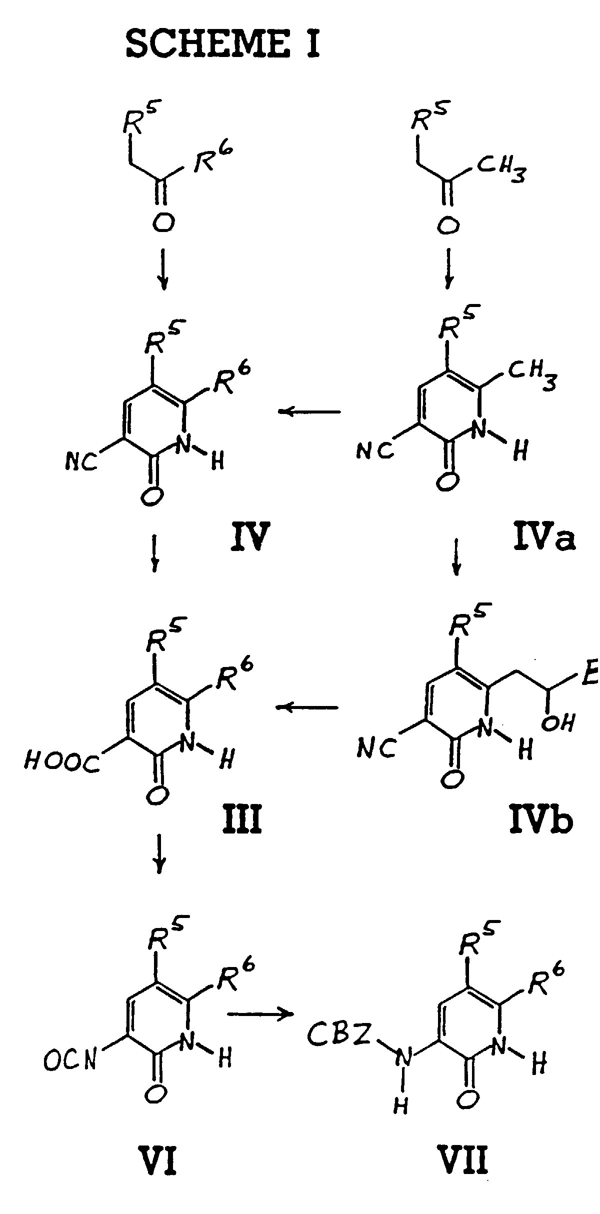

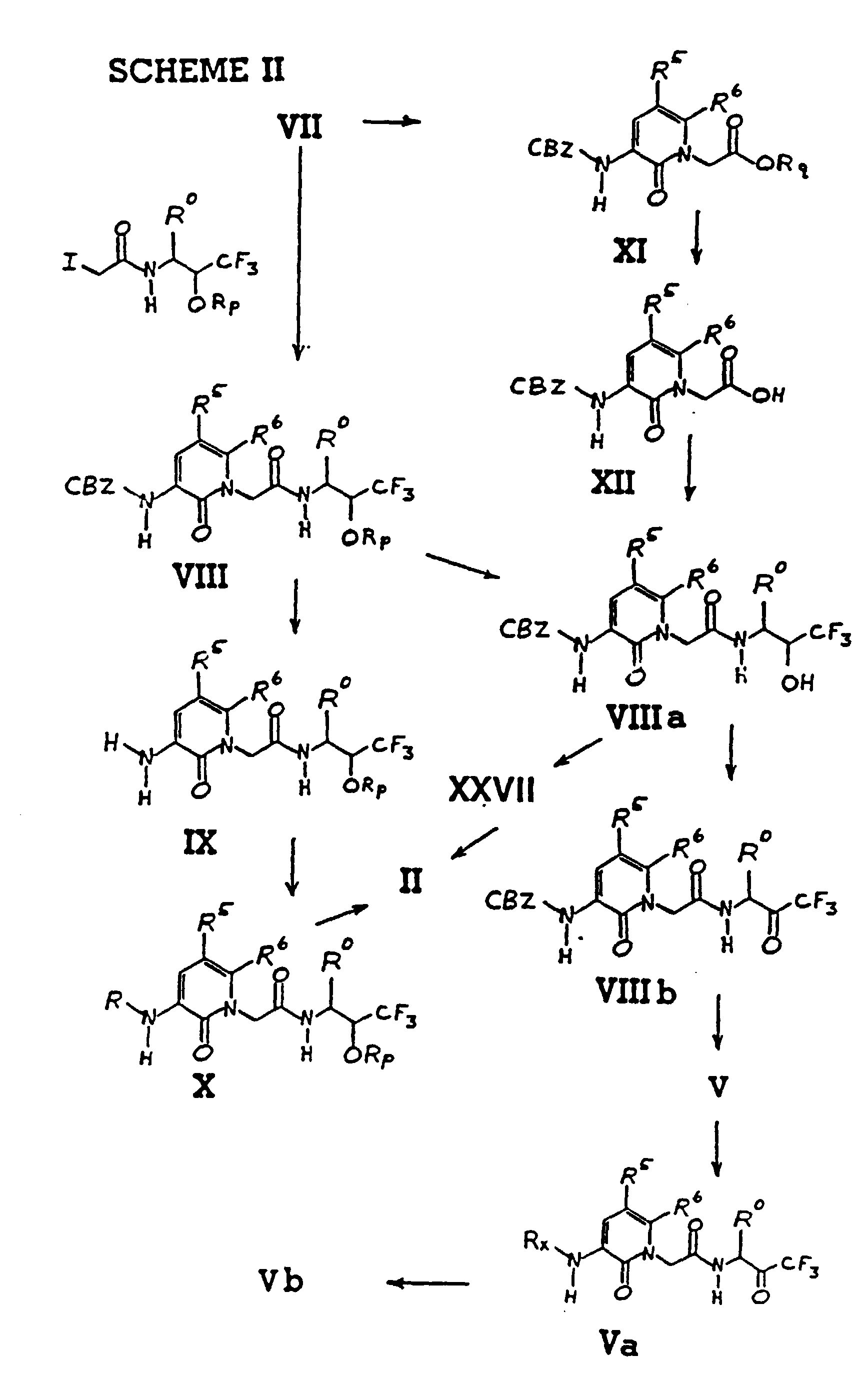

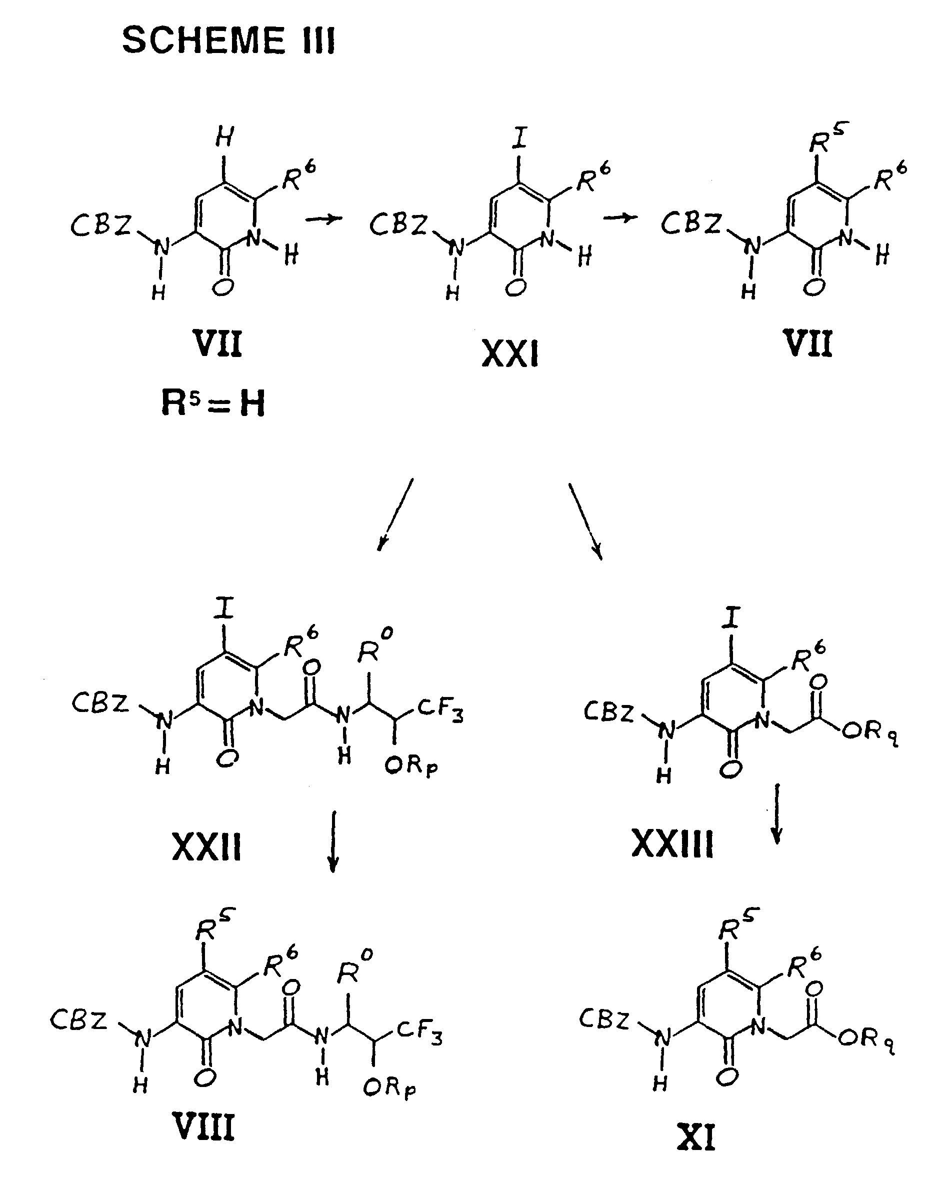

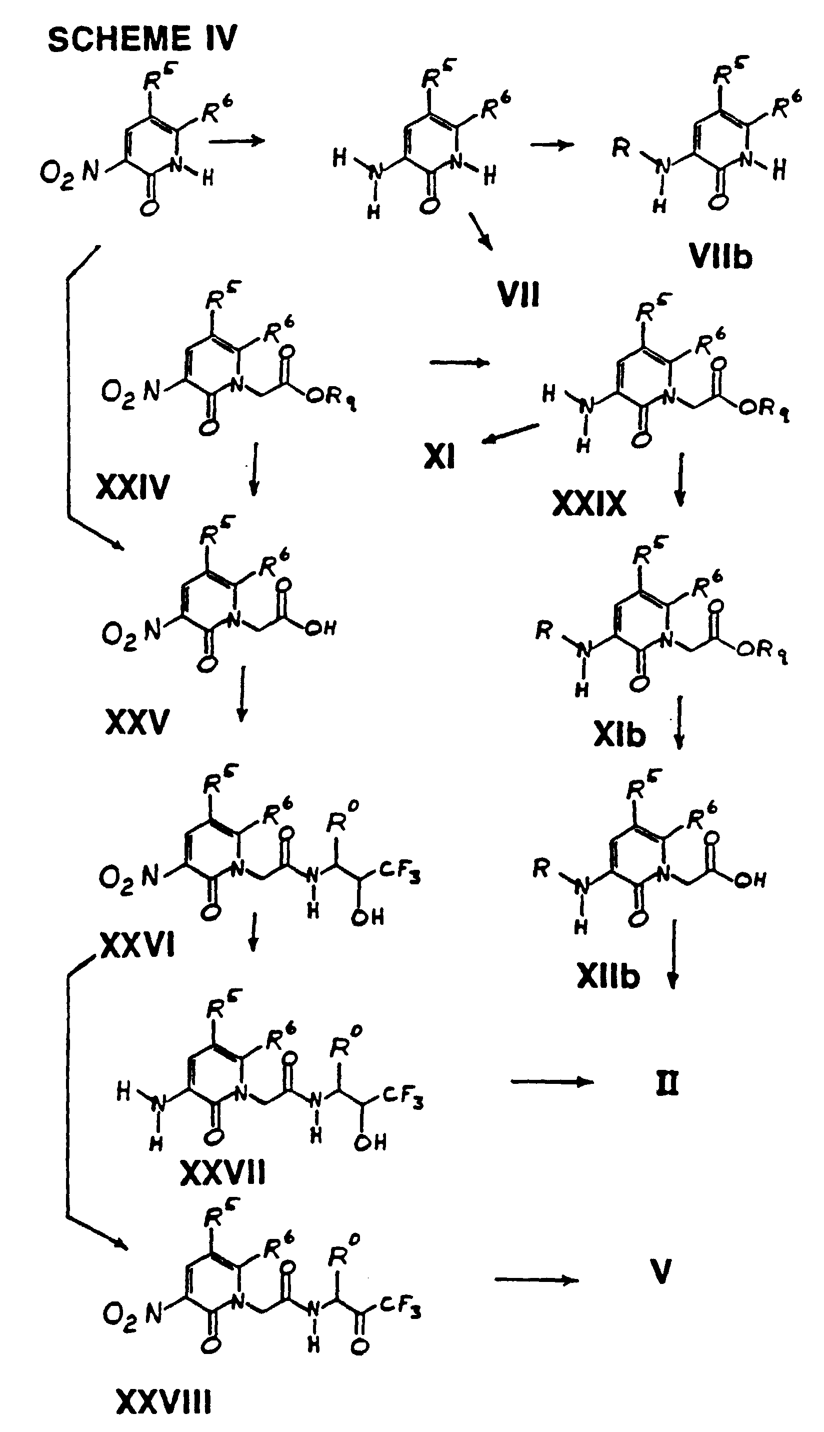

## Claims

1. A compound of formula I 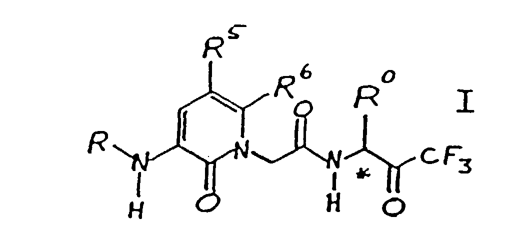 wherein:
R⁰ is (1-5C)alkyl;
R is 2,2,2-trifluoroethoxycarbonyl or 2,2,2-trifluoroethylaminocarbonyl; or
R is a sulfonyl group of formula D.W.SO₂- in which D.W-, taken together, is hydroxy, amino, di(lower alkyl)amino, 2,2,2-trifluoroethylamino, 3,3,3-trifluoropropyl, 2,2,2-trifluoroethyl or trifluoromethyl; or
W is a direct bond, imino, carbonylimino, oxycarbonylimino or iminocarbonylimino; and
D is as defined below; or
R is a group G as defined below;
The group D or G is (1-6C)alkyl, (3-6C)cycloalkyl, (3-6C)cycloalkyl-(1-3C)alkyl, aryl, aryl(1-3C)alkyl, heteroaryl or heteroaryl(1-3C)-alkyl wherein an alkyl carbon of G not bonded to the pyridone 3-amino nitrogen may bear an oxo group and wherein an aryl or heteroaryl moiety may bear one or more halogeno, nitro, methyl or trifluoromethyl groups and further wherein the group D or G may bear one or more substituents selected from a group consisting of hydroxy, lower alkoxy, lower acyloxy, COORa, CH₂COORa, CONRbRc, CH₂CONRbRC, COO(CH₂)₂NReRf, cyano, SO₂R¹, CONRdSO₂R¹, NReRf, NRgCHO, NRgCOR², NRgCOOR², NRhCQNRiRj, NRkSO₂R³, SO₂NRlRm, SO₂NRnCOR⁴ and P(O)(ORa)₂ in which
Q is oxygen or sulfur;
Ra-Rn are independently hydrogen, benzyl or lower alkyl; or, independently, a group NRbRc, NReRf, NRiRj or NRlRm is a cyclic radical selected from a group consisting of 1-pyrrolidinyl, piperidino, morpholino or 1-piperazinyl which may bear a lower alkyl substituent at the 4-position; or, independently, a group NReRf is a cyclic radical selected from a group consisting of 2-pyrrolidinon-1-yl, succinimido, oxazolidin-2-on-3-yl, 2-benzoxazolinon-3-yl, phthalimido and cis-hexahydrophthalimido; and
R¹-R⁴ are independently trifluoromethyl, (1-6C)alkyl, (3-6C)cycloalkyl, aryl or heteroaryl in which the aryl or heteroaryl may bear one or more substituents selected from a group consisting of lower alkyl, hydroxy, lower alkoxy, halogeno or trifluoromethyl;
Each of R⁵ and R⁶ is, independently, hydrogen or lower alkyl; or
One of R⁵ and R⁶ is hydrogen or methyl and the other of R⁵ and R⁶ is a radical of formula B.Y- in which
B is aryl or heteroaryl, which aryl or heteroaryl independently may bear one or more of the substituents defined for D or G or an aryl or heteroaryl moiety thereof;
Y is a direct bond, methylene, ethylene or trans-vinylene; and
provided that no aliphatic carbon is bonded to more than one nitrogen or oxygen, except as part of a cyclic ketal or where the nitrogen bears a carbonyl group; or,
for a compound of formula I which is acidic or basic, a pharmaceutically acceptable salt thereof.

2. A compound as claimed in Claim 1 wherein
R⁰ is ethyl or isopropyl;
D.W-, taken together, is amino, 2,2,2-trifluoroethylamino or 2,2,2-trifluoroethyl;
W is a direct bond or imino;
G is (1-3C)alkyl, aryl(1-C)alkyl or heteroaryl(1-2C)alkyl which may bear one or more substituents as defined in Claim 1 for G or a part thereof;
(1-6C)alkyl is methyl, ethyl, propyl, isopropyl, butyl, isobutyl, t-butyl, pentyl, 3-methylbutyl, 1-ethylpropyl, hexyl or 4-methylpentyl; (3-6C)cycloalkyl is cyclopropyl, cyclopentyl or cyclohexyl; the (1-3C)alkyl portion of (3-6C)cycloalkyl-(1-3C)alkyl, aryl(1-3C)alkyl or heteroaryl(1-3C)alkyl is methylene, ethylene or trimethylene; aryl is phenyl, indenyl or naphthyl; heteroaryl is furyl, imidazolyl, tetrazolyl, pyridyl (or its N-oxide), thienyl, pyrimidinyl (or its N-oxide), indolyl or quinolinyl (or its N-oxide); lower alkyl is methyl, ethyl, propyl, isopropyl, butyl, isobutyl or t-butyl; lower acyloxy is acetoxy; lower alkoxy is methoxy, ethoxy, propoxy, isoproxy or t-butoxy; halogeno is bromo, chloro or fluoro;
COORa is carboxy or methoxycarbonyl; NRgCHO is formylamino; NRgCOR² is acetylamino or trifluoroacetylamino; and CONRdSO₂R¹ is N-phenylsulfonylcarbamoyl or N-(4-chlorophenylsulfonyl)carbamoyl.

3. A compound as claimed in Claim 1 or 2 wherein R⁰ is isopropyl; D is methyl, ethyl, isopropyl, tert-butyl, cyclohexyl, phenyl, benzyl, phenethyl, pyridyl, thienyl, 5-tetrazolyl, thiazolyl, quinolinyl, pyridylmethyl, thenyl, 5-tetrazolylmethyl, 2-(pyridyl)ethyl, 2-(thienyl)ethyl or 2-(thiazolyl)ethyl wherein the phenyl or heteroaryl group may bear one or two halogeno or methyl groups and further wherein the group D may bear a substituent selected from hydroxy, methoxy, t-butoxy, acetoxy, pivaloyloxy, carboxy, methoxycarbonyl, ethoxycarbonyl, carbamoyl, dimethylcarbamoyl, 2-(dimethylamino)ethoxycarbonyl, cyano, methylsulfonyl, phenylsulfonyl, N-methylsulfonylcarbamoyl, N-phenylsulfonylcarbamoyl, N-(4-chlorophenylsulfonyl)carbamoyl, methylsulfonylamino, amino, dimethylamino, oxazolidin-2-on-3-yl, acetylamino, trifluoroacetylamino, ureido, methylsulfonyl, sulfamoyl, dimethylphosphoryl and diethylphosphoryl; and G is methyl, ethyl, benzyl, phenethyl, pyridyl, pyridylmethyl, thenyl, 5-tetrazolylmethyl or 2-(pyridyl)ethyl, wherein an alkyl carbon may bear an oxo group and wherein the phenyl or heteroaryl group may bear one or two halogeno or methyl groups and further wherein the group G may bear a substituent selected from hydroxy, methoxy, acetoxy, carboxy, methoxycarbonyl, ethoxycarbonyl, carbamoyl, dimethylcarbamoyl, phenylcarbamoyl, pyridylcarbamoyl, methylsulfonylamino, amino, dimethylamino, acetylamino, nicotinoylamino and trifluoroacetylamino.

4. A compound as claimed in Claim 1, 2 or 3 wherein R is sulfo, aminosulfonyl, dimethylaminosulfonyl, 2,2,2-trifluoroethylaminosulfonyl, 2,2,2-trifluoroethylsulfonyl, trifluoromethylsulfonyl, methylsulfonyl (which may bear a methoxycarbonyl, carboxy or ethylsulfonyl substituent), methylaminosulfonyl, isopropylaminosulfonyl, butylsulfonyl, butylaminosulfonyl, tert-butylaminosulfonyl, cyclohexylaminosulfonyl, phenylsulfonyl (in which the phenyl may bear a chloro, nitro, amino, acetylamino, trifluoroacetylamino, methoxy, carboxy, N-(4-chlorophenylsulfonyl)carbamoyl or methylsulfonylamino substituent at the 3- or 4-position), anilino, pyridylsulfonyl, quinolinylsulfonyl, benzylsulfonyl (in which the phenyl ring may bear a nitro or amino substituent at the 3- or 4-position), pyridylmethyl-sulfonyl, 2-(pyridyl)ethylsulfonyl or benzylaminosulfonyl.

5. A compound as claimed in any one of Claims 1-4 in which R⁵ is hydrogen and R⁶ is hydrogen.

6. A compound as claimed in any one of Claims 1-4 in which R⁵ is benzyl, the phenyl ring of which may bear a 3-fluoro, 4-fluoro, 4-trifluoromethyl, 4-methoxycarbonyl, 3-acetoxy, 3-hydroxy, 3-pivaloyloxy, 4-hydroxy, 4-pivaloyloxy, 3-trifluoroacetylamino or 3-amino substituent; and R⁶ is hydrogen.

7. A compound as claimed in any one of Claims 1-4 in which R⁵ is hydrogen; and R⁶ is 2-furyl, 2-thienyl, 3-pyridyl or phenyl in which the phenyl may bear one or two halogeno, trifluoromethyl, methyl, hydroxy, methoxy, tert-butoxy, methoxycarbonyl or carboxy substituents.

8. A compound as claimed in Claim 7 wherein R⁶ is phenyl, 4-fluorophenyl or 2-thienyl.

9. A compound as claimed in Claim in which R⁰ is isopropyl; R⁵ is hydrogen; R⁶ is phenyl; and R is 4-acetylaminophenylsulfonyl, 4-[N-(4-chlorophenylsulfonyl)carbamoyl]phenylsulfonyl, 4-[N-(2-methylphenylsulfonyl)carbamoyl]phenylsulfonyl or 2,2,2-trifluoroethoxycarbonyl.

10. A salt as claimed in Claim 1 selected from
(a) for an acidic compound of formula I, an alkalai metal salt, an alkaline earth metal salt, an aluminum salt, an ammonium salt, or a salt made from an organic base which affords a pharmaceutically acceptable cation; and
(b) for a basic compound of formula I, an acid-addition salt made with an acid which provides a pharmaceutically acceptable anion.

11. A method of making a compound of formula I, or a pharmaceutically acceptable salt thereof, as claimed in any one of Claims 1-10 which is characterized by:
(A) Oxidizing a corresponding alcohol of formula II; 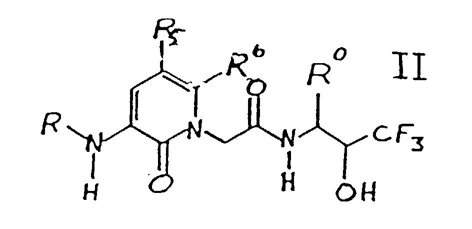
(B) For a compound of formula I which bears a hydroxy substituent on an aryl or heteroaryl group, cleaving the alkyl ether or acyloxy ester of a corresponding compound of formula I which bears a lower alkoxy or lower acyloxy substituent on an aryl or heteroaryl group;
(C) For a compound of formula I wherein R is 2,2,2-trifluoroethoxycarbonyl or 2,2,2-trifluoroethylaminocarbonyl,
acylation of a corresponding amine of formula V 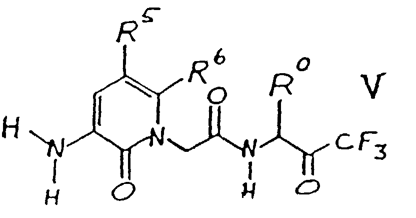 with 2,2,2-trifluoroethyl chloroformate or 2,2,2-trifluoroethyl isocyanate;
(D) For a compound of formula I wherein R is a sulfonyl group of formula D.W.SO²-, sulfonylation of a corresponding amine of formula V with a corresponding sulfonic acid of formula D.W.SO₂.OH, or an activated derivative thereof;
(E) For a compound of formula I wherein R is a group G, substitution of the group X of a corresponding compound of formula G-X, wherein X is a conventional leaving group, such as for example halogeno, methylsulfonyloxy, trifluoromethylsulfonyloxy or diazonium, with a corresponding amine of formula V;
(F) For a compound of formula I which bears a group of formula COORa in which Ra is hydrogen (a carboxy group), decomposing the ester group of a corresponding ester made with a conveniently removed acid protecting group;
(G) For a compound of formula I which contains an amino N-H residue, removal by using a conventional method of the nitrogen protecting group of a corresponding compound bearing a conventional nitrogen protecting group, including for a compound of formula I wherein R is G, the removal of an activating/protecting group Rx from a corresponding compound of formula Vb; 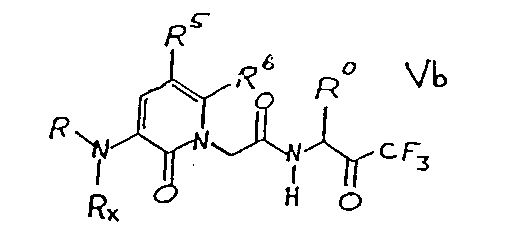
(H) For a compound of formula I bearing a moiety of formula COORa, CH₂COORa, CONRbRc, CH₂CONRbRC, COO(CH₂)₂NReRf or CONRdSO₂R¹, acylation of a corresponding compound of formula HORa, HNRbRc, HO(CH₂)₂NReRf or HNRdSO₂R¹ with a corresponding acid of formula I bearing a moiety of formula COORa in which Ra is hydrogen, or an activated derivative thereof;
(I) For a compound of formula I bearing a lower acyloxy group or a group of formula NRgCHO, NRgCOR², NRgCOOR², NRhCQNRiRj or NRkSO₂R³, acylation or sulfonylation of a corresponding compound of formula I bearing a hydroxy group or an amino group of formula NHRg, NHRh or NHRk (i.e. an amino group of formula NReRf is which Re is hydrogen and Rf is Rg, Rh or Rk) with an activated derivative of a corresponding acid of formula HOCHO, HOCOR², HOCOOR², HOCQNRiRj (including an isocyanate or isothiocyanate) or HOSO₂R³, respectively, using a conventional method;
(J) For a compound of formula I which bears a heteroaryl N-oxide group, oxidation of a corresponding compound of formula I which bears a heteroaryl group using a conventional oxidant; or
(K) For a compound of formula I which bears a primary amino group, reduction of a corresponding compound bearing a nitro group using a conventional reducing method; and
whereafter, for any of the above procedures, when a pharmaceutically acceptable salt of an acidic or basic compound of formula I is required, by reacting the acidic or basic form of such a compound of formula I with a base or acid affording a physiologically acceptable counterion or by any other conventional procedure; and
wherein each of R, R⁰, R⁵, R⁶, D, W, G, Ra-Rn, R¹-R³ and Q, except where more particularly described, has the meaning defined in any one of Claims 1-10.

12. A compound of formula II, wherein R, R⁰, R⁵ and R⁶ are defined as in Claim 1, or a salt thereof.

13. A compound of formula Vb, wherein R has a value defined for G in Claim 1; R⁰, R⁵ and R⁶ are defined as in Claim 1; and Rx is a group which protects and activates a primary amino group for substitution, or a salt thereof.

14. A pharmaceutical composition comprising a compound as defined in Claim 1, or a pharmaceutically acceptable salt thereof, and a pharmaceutically acceptable diluent or carrier.

## Patentansprüche

1. Verbindung mit der Formel I: 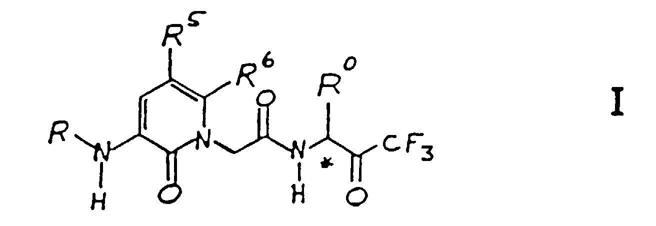 für die folgendes gilt:
R⁰ ist (1-5C)Alkyl,
R ist 2,2,2-Trifluorethoxycarbonyl oder 2,2,2-Trifluorethylaminocarbonyl, oder
R ist eine Sulfonyl-Gruppe mit der Formel D.W.SO₂-, wobei D.W- zusammengenommen für Hydroxy, Amino, Di(niederalkyl)amino, 2,2,2-Trifluorethylamino, 3,3,3-Trifluorpropyl, 2,2,2-Trifluorethyl oder Trifluormethyl steht, oder
W ist eine Direktbindung, Imino, Carbonylimino, Oxycarbonylimino oder Iminocarbonylimino; und
D bedeutet das unten definierte, oder
R ist eine unten definierte Gruppe G,
die Gruppe D oder G ist (1-6C)Alkyl, (3-6C)Cycloalkyl, (3-6C)Cycloalkyl-(1-3C)alkyl, Aryl, Aryl(1-3C)alkyl, Heteroaryl oder Heteroaryl(1-3C)alkyl, wobei ein Alkyl-Kohlenstoff von G, der nicht an den 3-Amino-Stickstoff des Pyridons gebunden ist, eine Oxo-Gruppe tragen kann, und ein Aryl- oder Heteroaryl-Rest eine oder mehrere Halogen-, Nitro-, Methyl- oder Trifluormethyl-Gruppen tragen kann und wobei die Gruppen D oder G ferner einen oder mehrere Substituenten tragen können, die unter Hydroxy, Niederalkoxy, Niederacyloxy, COORa, CH₂COORa, CONRbRc, CH₂CONRbRc, COO(CH₂)₂NReRf, Cyano, SO₂R¹, CONRdSO₂R¹, NReRf, NRgCHO, NRgCOR², NRgCOOR², NRhCQNRiRj, NRkSO₂R³, SO₂NRlRm, SO₂NRnCOR⁴ und P(O) (ORa)₂ ausgewählt sind, wobei
Q Sauerstoff oder Schwefel ist,
Ra - Rn unabhängig voneinander folgendes bedeuten: Wasserstoff, Benzyl oder Niederalkyl, oder, unabhängig voneinander, ist eine Gruppe NRbRc, NReRf, NRiRj oder NRlRm ein cyclischer Rest, der unter 1-Pyrrolidinyl, Piperidino, Morpholino oder 1-Piperazinyl, das einen Niederalkyl-Substituenten in Position 4 tragen kann, ausgewählt ist, oder, unabhängig voneinander, ist eine Gruppe NReRf ein cyclischer Rest, der unter 2-Pyrrolidinon-1-yl, Succinimido, Oxazolidin-2-on-3-yl, 2-Benzoxazolinon-3-yl, Phthalimido und cis-Hexahydrophthalimido ausgewählt ist, und
R¹-R⁴ sind unabhängig voneinander Trifluormethyl, (1-6C)Alkyl, (3-6C)Cycloalkyl, Aryl oder Heteroaryl, wobei das Aryl oder Heteroaryl einen oder mehrere Substituenten tragen kann, die unter Niederalkyl, Hydroxy, Niederalkoxy, Halogen oder Trifluormethyl ausgewählt sind,
R⁵ und R⁶ sind jeweils unabhängig voneinander Wasserstoff oder Niederalkyl, oder
einer der Substituenten R⁵ und R⁶ ist Wasserstoff oder Methyl und der andere der Substituenten R⁵ und R⁶ ist eine Rest mit der Formel B.Y-, wobei
B Aryl oder Heteroaryl ist, welches Aryl oder Heteroaryl unabhängig voneinander einen oder mehrere der für D oder G oder einen Aryl- oder Heteroaryl-Rest davon definierten Substituenten tragen kann,
Y ist eine Direktbindung, Methylen, Ethylen oder trans-Vinylen,
mit der Maßgabe, daß kein aliphatischer Kohlenstoff an mehr als einen Stickstoff oder Sauerstoff gebunden ist, ausgenommen als Teil eines cyclischen Ketals oder wenn der Stickstoff eine Carbonyl-Gruppe trägt, oder
ein pharmazeutisch geeignetes Salz davon, wenn die Verbindung mit der Formel I sauer oder basisch ist.

2. Verbindung nach Anspruch 1, in der:
R⁰ Ethyl oder Isopropyl ist,
D.W- zusammengenommen Amino, 2,2,2-Trifluorethylamino oder 2,2,2-Trifluorethyl ist,
W eine Direktbindung oder Imino ist,
G (1-3C)Alkyl, Aryl(1-3C)alkyl oder Heteroaryl(1-2C)alkyl ist, das einen oder mehrere in Anspruch 1 für G oder einen Teil davon definierte Substituenten tragen kann,
(1-6C)Alkyl Methyl, Ethyl, Propyl, Isopropyl, Butyl, Isobutyl, t-Butyl, Pentyl, 3-Methylbutyl, 1-Ethylpropyl, Hexyl oder 4-Methylpentyl ist, (3-6C)Cycloalkyl Cyclopropyl, Cyclopentyl oder Cyclohexyl ist, der (1-3C)Alkyl-Teil von (3-6C)Cycloalkyl(1-3C)alkyl, Aryl(1-3C)alkyl oder Heteroaryl(1-3C)alkyl Methylen, Ethylen oder Trimethylen ist, Aryl Phenyl, Indenyl oder Naphthyl ist, Heteroaryl Furyl, Imidazolyl, Tetrazolyl, Pyridyl (oder dessen N-Oxid), Thienyl, Pyrimidinyl (oder dessen N-Oxid), Indolyl oder Chinolinyl (oder dessen N-Oxid) ist, Niederalkyl Methyl, Ethyl, Propyl, Isopropyl, Butyl, Isobutyl oder t-Butyl ist, Niederacyloxy Acetoxy ist, Niederalkoxy Methoxy, Ethoxy, Propoxy, Isopropoxy oder t-Butoxy ist, Halogen Brom, Chlor oder Fluor ist,
COORa Carboxy oder Methoxycarbonyl ist, NRgCHO Formylamino ist, NRgCOR² Acetylamino oder Trifluoracetylamino ist und CONRdSO₂R¹ N-Phenylsulfonylcarbamoyl oder N-(4-chlorphenylsulfonyl)carbamoyl ist.

3. Verbindung nach Anspruch 1 oder 2, wobei
R⁰ Isopropyl ist, D Methyl, Ethyl, Isopropyl, tert.-Butyl, Cyclohexyl, Phenyl, Benzyl, Phenethyl, Pyridyl, Thienyl, 5-Tetrazolyl, Thiazolyl, Chinolinyl, Pyridylmethyl, Thenyl, 5-Tetrazolylmethyl, 2-(Pyridyl)ethyl, 2-(Thienyl)ethyl oder 2-(Thiazolyl)ethyl ist, wobei die Phenyl- oder Heteroaryl-Gruppe eine oder zwei Halogen- oder Methyl-Gruppen tragen kann und wobei ferner die Gruppe D einen Substituenten tragen kann, der unter Hydroxy, Methoxy, t-Butoxy, Acetoxy, Pivaloyloxy, Carboxy, Methoxycarbonyl, Ethoxycarbonyl, Carbamoyl, Dimethylcarbamoyl, 2-(Dimethylamino)ethoxycarbonyl, Cyano, Methylsulfonyl, Phenylsulfonyl, N-Methylsulfonylcarbamoyl, N-Phenylsulfonylcarbamoyl, N-(4-Chlorphenylsulfonyl)carbamoyl, Methylsulfonylamino, Amino, Dimethylamino, Oxazolidin-2-on-3-yl, Acetylamino, Trifluoracetylamino, Ureido, Methylsulfonyl, Sulfamoyl, Dimethylphosphoryl und Diethylphosphoryl ausgewählt ist, und G für Methyl, Ethyl, Benzyl, Phenethyl, Pyridyl, Pyridylmethyl, Thenyl, 5-Tetrazolylmethyl oder 2-(Pyridyl)ethyl steht, wobei ein Alkyl-Kohlenstoff eine Oxo-Gruppe tragen kann und wobei die Phenyl- oder Heteroaryl-Gruppe eine oder zwei Halogen- oder Methyl-Gruppen tragen kann und wobei ferner die Gruppe G einen Substituenten tragen kann, der unter Hydroxy, Methoxy, Acetoxy, Carboxy, Methoxycarbonyl, Ethoxycarbonyl, Carbamoyl, Dimethylcarbamoyl, Phenylcarbamoyl, Pyridylcarbamoyl, Methylsulfonamino, Amino, Dimethylamino, Acetylamino, Nicotinoylamino und Trifluoracetylamino ausgewählt ist.

4. Verbindung nach Anspruch 1, 2 oder 3, wobei
R für Sulfo, Aminosulfoyl, Dimethylaminosulfonyl, 2,2,2-Trifluorethylaminosulfonyl, 2,2,2-Trifluorethylsulfonyl, Trifluormethylsulfonyl, Methylsulfonyl (das einen Methoxycarbonyl-, Carboxy- oder Ethylsulfonyl-Substituenten tragen kann), Methylaminosulfonyl, Isopropylaminosulfonyl, Butylsulfonyl, Butylaminosulfonyl, tert.-Butylaminosulfonyl, Cyclohexylaminosulfonyl, Phenylsulfonyl (wobei das Phenyl in der Position 3 oder 4 einen Chlor-, Nitro-, Amino-, Acetylamino-, Trifluoracetylamino-, Methoxy-, Carboxy-, N-(4-Chlorphenylsulfonyl)carbamoyl- oder Methylsulfonylamino-Substituenten tragen kann), Anilino, Pyridylsulfonyl, Chinolinylsulfonyl, Benzylsulfonyl (wobei der Phenyl-Ring in der Position 3 oder 4 einen Nitro- oder Amino-Substituenten tragen kann), Pyridylmethylsulfonyl, 2-(Pyridyl)ethylsulfonyl oder Benzylaminosulfonyl steht.

5. Verbindung nach einem der Ansprüche 1 bis 4, wobei R⁵ Wasserstoff und R⁶ Wasserstoff ist.

6. Verbindung nach einem der Ansprüche 1 bis 4, wobei R⁵ Benzyl ist, dessen Phenyl-Ring einen 3-Fluor-, 4-Fluor-, 4-Trifluormethyl-, 4-Methoxycarbonyl-, 3-Acetoxy-, 3-Hydroxy-, 3-Pivaloyloxy-, 4-Hydroxy-, 4-Pivaloyloxy-, 3-Trifluoracetylamino- oder 3-Amino-Substituenten tragen kann, und R⁶ Wasserstoff ist.

7. Verbindung nach einem der Ansprüche 1 bis 4, wobei R⁵ Wasserstoff ist und R⁶ 2-Furyl, 2-Thienyl, 3-Pyridyl oder Phenyl ist, wobei das Phenyl einen oder zwei Halogen-, Trifluormethyl-, Methyl-, Hydroxy-, Methoxy-, tert.Butoxy-, Methoxycarbonyl- oder Carboxy-Substituenten tragen kann.

8. Verbindung nach Anspruch 7, wobei R⁶ Phenyl, 4-Fluorphenyl oder 2-Thienyl ist.

9. Verbindung nach Anspruch 1, wobei R⁰ Isopropyl ist, R⁵ Wasserstoff ist, R⁶ Phenyl ist und R 4-Acetylaminophenylsulfonyl, 4-[N-(4-Chlorphenylsulfonyl)carbamoyl]phenylsulfonyl, 4-[N-(2-Methylphenylsulfonyl)carbamoyl]phenylsulfonyl oder 2,2,2-Trifluorethoxycarbonyl ist.

10. Salz nach Anspruch 1, das unter folgenden ausgewählt ist:
(a) für eine saure Verbindung mit der Formel I: einem Alkalimetallsalz, einem Erdalkalimetallsalz, einem Aluminiumsalz, einem Ammoniumsalz oder einem Salz, das mit einer organischen Base hergestellt wird, die ein pharmazeutisch geeignetes Kation liefert, und
(b) für eine basische Verbindung mit der Formel I: einem Säureadditionssalz, das mit einer Säure hergestellt wird, die ein pharmazeutisch geeignetes Anion liefert.

11. Verfahren zur Herstellung einer Verbindung mit der Formel I, oder eines pharmazeutisch geeigneten Salzes davon, nach einem der Ansprüche 1 bis 10, das dadurch **gekennzeichnet** ist,
daß:
(A) ein entsprechender Alkohol mit der Formel II: 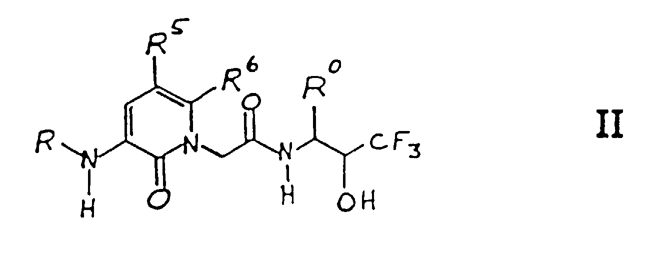 oxidiert wird,
(B) zur Herstellung einer Verbindung mit der Formel I, die einen Hydroxy-Substituenten an einer Aryl- oder Heteroaryl-Gruppe trägt, der Alkylether oder Acyloxyester einer entsprechenden Verbindung mit der Formel I, die einen Niederalkoxy- oder Niederacyloxy-Substituenten an einer Aryl- oder Heteroaryl-Gruppe trägt, gespalten wird,
(C) zur Herstellung einer Verbindung mit der Formel I, in der R 2,2,2-Trifluorethoxycarbonyl oder 2,2,2-Trifluorethylaminocarbonyl ist, ein entsprechendes Amin mit der Formel V: 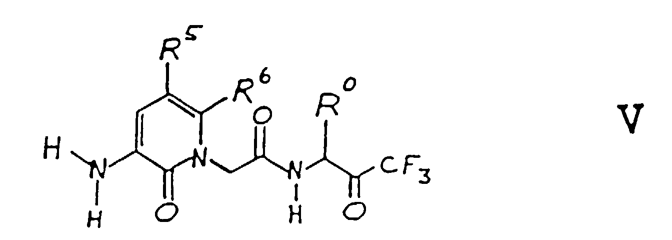 mit Chlorameisensäure-2,2,2-trifluorethylester oder 2,2,2-Trifluorethylisocyanat acyliert wird,
(D) zur Herstellung einer Verbindung mit der Formel I, in der R eine Sulfonyl-Gruppe mit der Formel D.W.SO²- ist, ein entsprechendes Amin mit der Formel V mit einer entsprechenden Sulfonsäure mit der Formel D.W.SO₂.OH, oder einem aktivierten Derivat davon, sulfoniert wird,
(E) zur Herstellung einer Verbindung mit der Formel I, in der R eine Gruppe G ist, die Gruppe X einer entsprechenden Verbindung mit der Formel G-X, in der X für eine übliche Austrittsgruppe steht, beispielsweise Halogen, Methylsulfonyloxy, Trifluormethylsulfonyloxy oder Diazonium, mit einem entsprechenden Amin mit der Formel V substituiert wird,
(F) zur Herstellung einer Verbindung mit der Formel I, die eine Gruppe mit der Formel COORa trägt, in der Ra Wasserstoff ist (eine Carboxy-Gruppe), die Ester-Gruppe eines entsprechenden Esters, der mit einer geeignet entfernten Säureschutzgruppe hergestellt wurde, zersetzt wird,
(G) zur Herstellung einer Verbindung mit der Formel I, die einen Amino-N-H-Rest enthält, unter Anwendung eines üblichen Verfahrens die Stickstoff-Schutzgruppe aus einer entsprechenden Verbindung entfernt wird, die eine übliche Stickstoff-Schutzgruppe enthält, wobei die Entfernung einer Aktivierungs/Schutzgruppe Rx aus einer entsprechenden Verbindung mit der Formel Vb: 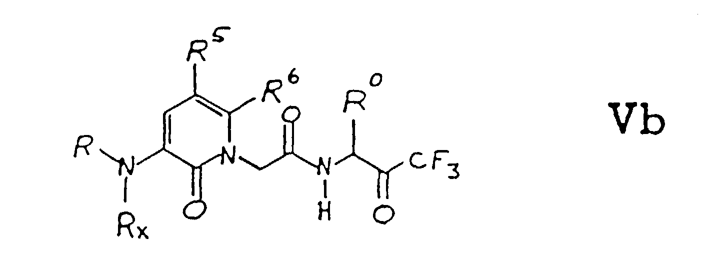 zur Herstellung einer Verbindung mit der Formel I, in der R G ist, eingeschlossen ist,
(H) zur Herstellung einer Verbindung mit der Formel I, die einen Rest mit der Formel COORa, CH₂COORa, CONRbRc, CH₂CONRbRc, COO(CH₂)₂NReRf oder CONRdSO₂R¹ trägt, eine entsprechende Verbindung mit der Formel HORa, HNRbRc, HO(CH₂)₂NReRf oder HNRdSO₂R¹ mit einer entsprechenden Säure mit der Formel I, die einen Rest mit der Formel COORa trägt, in der Ra Wasserstoff ist, oder einem aktivierten Derivat davon, acyliert wird,
(I) zur Herstellung einer Verbindung mit der Formel I, die eine Niederacyloxy-Gruppe oder eine Gruppe mit der Formel NRgCHO, NRgCOR², NRgCOOR², NRhCQNRiRj oder NRkSO₂R³ trägt, eine entsprechende Verbindung mit der Formel I, die eine Hydroxy-Gruppe oder eine Amino-Gruppe mit der Formel NHRg, NHRh oder NHRk (d.h. eine Amino-Gruppe mit der Formel NReRf, in der Re Wasserstoff ist und Rf Rg, Rh oder Rk ist) trägt, mit einem aktivierten Derivat einer entsprechenden Säure mit der Formel HOCHO, HOCOR², HOCOOR², HOCQNRiRj (einschließlich eines Isocyanats oder Isothiocyanats) bzw. HOSO₂R³ unter Anwendung eines üblichen Verfahrens acyliert oder sulfoniert wird,
(J) zur Herstellung einer Verbindung mit der Formel I, die eine Heteroaryl-N-oxid-Gruppe trägt, eine entsprechende Verbindung mit der Formel I, die eine Heteroaryl-Gruppe trägt, unter Verwendung eines üblichen Oxidationsmittels oxidiert wird, oder
(K) zur Herstellung einer Verbindung mit der Formel I, die eine primäre Amino-Gruppe trägt, eine entsprechende Verbindung, die eine Nitro-Gruppe trägt, unter Anwendung eines üblichen Reduktionsverfahrens reduziert wird,
im Anschluß daran, bei jedem der obigen Verfahren, wenn ein pharmazeutisch geeignetes Salz einer sauren oder basischen Verbindung mit der Formel I erforderlich ist, die saure oder basische Form einer derartigen Verbindung mit der Formel I mit einer Base oder Säure, die ein physiologisch geeignetes Gegenion liefert oder nach jedem beliebigen anderen üblichen Verfahren umgesetzt wird,
wobei R, R⁰, R⁵, R⁶, D, W, G. Ra-Rn, R¹-R³ und Q, sofern sie nicht besonders angegeben sind, die in einem der Ansprüche 1 bis 10 definierten Bedeutungen haben.

12. Verbindung mit der Formel II, in der R, R⁰, R⁵ und R⁶ wie in Anspruch definiert sind, oder ein Salz davon.

13. Verbindung mit der Formel Vb, in der R einen für G in Anspruch 1 definierten Wert hat, R⁰, R⁵ und R⁶ wie in Anspruch 1 definiert sind und Rx eine Gruppe ist, welche eine primäre Amino-Gruppe zur Substitution schützt und aktiviert, oder ein Salz davon.

14. Pharmazeutische Zusammensetzung, die eine Verbindung nach Anspruch 1, oder ein pharmazeutisch geeignetes Salz davon, und einen pharmazeutisch geeigneten Streckstoff oder Trägerstoff enthält.

## Revendications

1. Composé de formule I 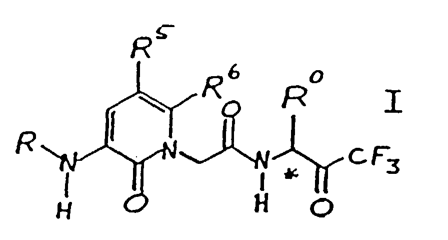 dans laquelle :
R⁰ représente un groupe alkyle en C₁ à C₅ ;
R représente un groupe 2,2,2-trifluoroéthoxycarbonyle ou 2,2,2-trifluoroéthylaminocarbonyle ; ou
R représente un groupe sulfonyle de formule D.W.SO₂- dans laquelle D. et W., pris conjointement, représentent un groupe hydroxy, amino, di(alkyle inférieur)amino, 2,2,2-trifluoroéthylamino, 3,3,3-trifluoropropyle, 2,2,2-trifluoréthyle ou trifluorométhyle ; ou
W représente une liaison directe, un groupe imino, carbonylimino, oxycarbonylimino ou iminocarbonylimino ; et
D répond à la définition ci-dessous ; ou
R représente un groupe G répondant à la définition ci-dessous ;
le groupe D ou G représente un groupe alkyle en C₁ à C₆, cycloalkyle en C₃ à C₆, (cycloalkyle en C₃ à C₆)alkyle en C₁ à C₃, aryle, aryl-(alkyle en C₁ à C₃), hétéroaryle ou hétéroaryl-(alkyle en C₁ à C₃) dans lequel un atome de carbone du groupe alkyle de G non lié à l'atome d'azote du groupe 3-amino de la pyridone peut porter un groupe oxo, et dans lequel un groupement aryle ou hétéroaryle peut porter un ou plusieurs groupes halogéno, nitro, méthyle ou trifluorométhyle et, en outre, le groupe D ou G peut porter un ou plusieurs substituants choisis dans le groupe consistant en des substituants hydroxy, alkoxy inférieur, acyloxy inférieur, COORa, CH₂ROORa, CONRbRc, CH₂CONRbRc, COO(CH₂)₂NReRf, cyano, SO₂R¹, CONRdSO₂R¹, NReRf, NRgCHO, NRgCOR², NRgCOOR², NRhCQNRiRj, NRkSO₂R³, SO₂NR₁Rm, SO₂NRnCOR⁴ et P(O) (ORa) ₂ dans lesquels
Q représente l'oxygène ou le soufre ;
Ra-Rn représente indépendamment l'hydrogène, un groupe benzyle ou alkyle inférieur ; ou, indépendamment, un groupe NRbRc, NReRf, NRiRj ou NRlRm est un radical cyclique choisi dans le groupe consistant en les radicaux 1-pyrrolidinyle, pipéridino, morpholino et 1-pipérazinyle pouvant porter un substituant alkyle inférieur en position 4 ; ou, indépendamment, un groupe NReRf est un radical cyclique choisi dans le groupe consistant en des radicaux 2-pyrrolidinone-1-yle, succinimido, oxazolidine-2-one-3-yle, 2-benzoxazolinone-3-yle, phtalimido et cis-hexahydrophtalimido ; et
R¹-R⁴ représentent indépendamment des groupes trifluorométhyle, alkyle en C₁ à C₆, cycloalkyle en C₃ à C₆, aryle ou hétéroaryle dans lesquels le groupe aryle ou hétéroaryle peut porter un ou plusieurs substituants choisis dans le groupe consistant en des substituants alkyle inférieur, hydroxy, alkoxy inférieur, halogéno et trifluorométhyle ;
chacun des groupes R⁵ et R⁶ représente, indépendamment, l'hydrogène ou un groupe alkyle inférieur ; ou
un des groupes R⁵ et R⁶ représente l'hydrogène ou un groupe méthyle et l'autre des groupes R⁵ et R⁶ représente un radical de formule B.Y- dans laquelle
B représente un groupe aryle ou hétéroaryle, groupe aryle ou hétéroaryle qui peut porter indépendamment un ou plusieurs des substituants définis pour D ou G ou un de leurs groupements aryle ou hétéroaryle ;
Y représente une liaison directe, un groupe méthylène, éthylène ou trans-vinylène ; et
sous réserve qu'aucun atome de carbone aliphatique ne soit lié à plus d'un atome d'azote ou d'oxygène, sauf en tant qu'une partie d'un cétal cyclique ou lorsque l'atome d'azote porte un groupe carbonyle ; ou,
pour un composé de formule I qui est acide ou basique, un de ses sels pharmaceutiquement acceptables.

2. Composé suivant la revendication 1, dans lequel
R⁰ représente un groupe éthyle ou isopropyle ;
D et W-, pris conjointement, représentent un groupe amino, 2,2,2-trifluoréthylamino ou 2,2,2-trifluoréthyle ;
W représente une liaison directe ou un groupe d'imino ;
G représente un groupe alkyle en C₁ à C₃, aryl(alkyle en C₁ ou C₂) ou hétéroaryl-(alkyle en C₁ ou C₂) qui peut porter un ou plusieurs substituants répondant à la définition suivant la revendication 1 pour G ou une de ses parties ;
le groupe alkyle en C₁ à C₆ est un groupe méthyle, éthyle, propyle, isopropyle, butyle, isobutyle, tertio-butyle, pentyle, 3-méthylbutyle, 1-éthylpropyle, hexyle ou 4-méthylpentyle, le groupe cycloalkyle en C₃ à C₆ est un groupe cyclopropyle, cyclopentyle ou cyclohexyle ; la portion alkyle en C₁ à C₃ du groupe (cycloalkyle en C₃ à C₆)(alkyle en C₁ à C₃), aryl-(alkyle en C₁ à C₃) ou hétéroaryl(alkyle en C₁ à C₃) est un groupe méthylène, éthylène ou triméthylène ; le groupe aryle est un groupe phényle, indényle ou naphtyle ; le groupe hétéroaryle est un groupe furyle, imidazolyle, tétrazolyle, pyridyle (ou son N-oxyde), thiényle, pyrimidinyle (ou son N-oxyde), indolyle ou quinolinyle (ou son N-oxyde) le groupe alkyle inférieur est un groupe méthyle, éthyle, propyle, isopropyle, butyle, isobutyle ou tertio-butyle ; le groupe acyloxy inférieur est un groupe acétoxy ; le groupe alkoxy inférieur est un groupe méthoxy, éthoxy, propoxy, isopropoxy ou tertio-butoxy ; le groupe halogéno est un groupe bromo, chloro ou fluoro ;
le groupe COORa est un groupe carboxy ou méthoxycarbonyle ; le groupe NRgCHO est un groupe formylamino ; le groupe NRgCOR² est un groupe acétylamino ou trifluoracétylamino ; et le groupe CONRdSO₂R¹ est un groupe N-phénylsulfonylcarbamoyle ou N-(4-chlorophénylsulfonyl)carbamoyle.

3. Composé suivant la revendication 1 ou la revendication 2, dans lequel R⁰ représente un groupe isopropyle ; D représente un groupe méthyle, éthyle, isopropyle, tertiobutyle, cyclohexyle, phényle, benzyle, phénétyle, pyridyle, thiényle, 5-tétrazolyle, thiazolyle, quinolinyle, pyridylméthyle, thényle, 5-tétrazolylméthyle, 2-(pyridyl)éthyle, 2-(thiényl)éthyle ou 2-(thiazolyl)éthyle dans lequel le groupe phényle ou hétéroaryle peut porter un ou deux groupes halogéno ou méthyle et, en outre, le groupe D peut porter un substituant choisi entre les substituants hydroxy, méthoxy, t-butoxy, acétoxy, pivaloyloxy, carboxy, méthoxycarbonyle, éthoxycarbonyle, carbamoyle, diméthylcarbamoyle, 2-(diméthylamino)éthoxycarbonyle, cyano, méthylsulfonyle, phénylsulfonyle, N-méthylsulfonylcarbamoyle, N-phénylsulfonylcarbamoyle, N-(4-chlorophénylsulfonyl)carbamoyle, méthylsulfonylamino, amino, diméthylamino, oxazolidine-2-one-3-yle, acétylamino, trifluoracétylamino, uréido, méthylsulfonyle, sulfamoyle, diméthylphosphoryle et diéthylphosphoryle et G représente un groupe méthyle, éthyle, benzyle, phénéthyle, pyridyle, pyridylméthyle, thényle, 5-tétrazolylméthyle ou 2-(pyridyl)éthyle, dans lequel un atome de carbone de groupe alkyle peut porter un groupe oxo et le groupe phényle ou hétéroaryle peut porter un ou deux groupes halogéno ou méthyle et, en outre, le groupe G peut porter un substituant choisi entre les groupes hydroxy, méthoxy, acétoxy, carboxy, méthoxycarbonyle, éthoxycarbonyle, carbamoyle, diméthylcarbamoyle, phénylcarbamoyle, pyridylcarbamoyle, méthylsulfonylamino, amino, diméthylamino, acétylamino, nicotinoylamino et trifluoracétylamino.

4. Composé suivant la revendication 1, 2 ou 3, dans lequel R représente un groupe sulfo, aminosulfonyle, diméthylaminosulfonyle, 2,2,2-trifluoréthylaminosulfonyle, 2, 2,2-trifluoréthylsulfonyle, trifluorométhylsufolnyle, méthylsulfonyle (qui peut porter un substituant méthoxycarbonyle, carboxy ou éthylsulfonyle), méthylaminosulfonyle, isopropylaminosulfonyle, butylsulfonyle, butylaminosulfonyle, tertio-butylaminosulfonyle, cyclohexylaminosulfonyle, phénylsulfonyle (dans lequel le groupe phényle peut porter un substituant chloro, nitro, amino, acétylamino, trifluoracétylamino, méthoxy, carboxy, N-(4-chlorophénylsulfonyl)carbamoyle ou méthylsulfonylamino en position 3- ou 4-), anilino, pyridylsulfonyle, quinolinylsulfonyle, benzylsulfonyle (dans lequel le noyau phényle peut porter un substituant nitro ou amino en position 3- ou 4), pyridylméthylsulfonyle, 2-(pyridyl)éthylsulfonyle ou benzylaminosulfonyle.

5. Composé suivant l'une quelconque des revendications 1 à 4, dans lequel R⁵ représente l'hydrogène et R⁶ représente l'hydrogène.

6. Composé suivant l'une quelconque des revendications 1 à 4, dans lequel R⁵ représente un groupe benzyle, dont le noyau phényle peut porter un substituant 3-fluoro, 4-fluoro, 4-trifluorométhyle, 4-méthoxycarbonyle, 3-acétoxy, 3-hydroxy, 3-pivaloyloxy, 4-hydroxy, 4-pivaloyloxy, 3-trifluoracétylamino ou 3-amino ; et R⁶ représente l'hydrogène.

7. Composé suivant l'une quelconque des revendications 1 à 4, dans lequel R⁵ représente l'hydrogène ; et R⁶ représente un groupe 2-furyle 2-thiényle, 3-pyridyle ou phényle dans lequel le groupe phényle peut porter un ou deux substituants halogéno, trifluorométhyle, méthyle, hydroxy, méthoxy, tertio-butoxy, méthoxycarbonyle ou carboxy.

8. Composé suivant la revendication 7, dans lequel R⁶ représente un groupe phényle, 4-fluorophényle ou 2-thiényle.

9. Composé suivant la revendication 1, dans lequel R⁰ représente un groupe isopropyle ; R⁵ représente l'hydrogène ; R⁶ représente un groupe phényle et R représente un groupe 4-acétylaminophénylsulfonyle, 4- [N- (4-chlorophénylsulfonyl)-carbamoyl]phénylsulfonyle, 4-[N-(2-méthylphénylsulfonyl)carbamoyl]phénysulfonyle ou 2,2,2-trifluoréthoxycarbonyle.

10. Sel suivant la revendication 1, choisi entre
(a) pour un composé acide de formule I, un sel de métal alcalin, un sel de métal alcalino-terreux, un sel d'aluminium, un sel d'ammonium ou un sel préparé à partir d'une base organique qui donne un cation pharmaceutiquement acceptable ; et
(b) pour un composé basique de formule I, un sel d'addition d'acide préparé avec un acide qui donne un anion pharmaceutiquement acceptable.

11. Procédé de préparation d'un composé de formule I, ou d'un de ses sels pharmaceutiquement acceptables, suivant l'une quelconque des revendications 1 à 10, qui est caractérisé par :
(A) l'oxydation d'un alcool correspondant de formule II 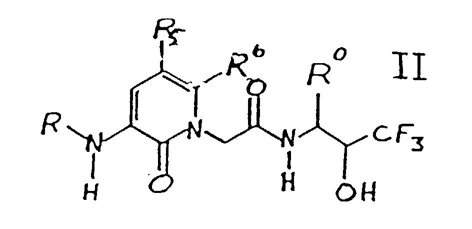
(B) pour un composé de formule I qui porte un substituant hydroxy sur un groupe aryle ou hétéroaryle, le clivage de l'éther alkylique ou de l'acyloxy-ester d'un composé correspondant de formule I qui porte un substituant alkoxy inférieur ou acyloxy inférieur sur un groupe aryle ou hétéroaryle ;
(C) pour un composé de formule I dans laquelle R représente un groupe 2,2,2-trifluoréthoxycarbonyle ou 2,2,2-trifluoréthylaminocarbonyle, l'acylation d'une amine correspondante de formule V 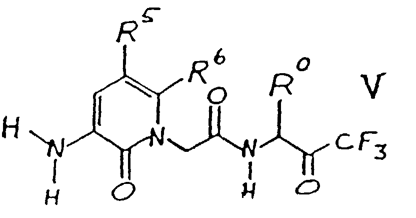 avec le chloroformiate de 2,2,2-trifluoréthyle ou l'isocyanate de 2,2,2-trifluoréthyle;
(D) pour un composé de formule I dans laquelle R représente un groupe sulfonyle de formule D.W.SO²-, la sulfonylation d'une amine correspondante de formule V avec un acide sulfonique correspondant de formule D.W.SO₂.OH, ou un de ses dérivés activés ;
(E) pour un composé de formule I, dans laquelle R représente un groupe G, la substitution du groupe X d'un composé correspondant de formule G-X, dans laquelle X représente un groupe partant classique, tel que par exemple un groupe halogéno, méthylsulfonyloxy, trifluorométhylsulfonyloxy ou diazonium, par une amine correspondante de formule V ;
(F) pour un composé de formule I qui porte un groupe de formule COORa dans laquelle Ra représente l'hydrogène (un groupe carboxy), la décomposition du groupe ester d'un ester correspondant préparé avec un groupe protecteur d'acide convenablement éliminé ;
(G) pour un composé de formule I qui contient un résidu N-H de groupe amino, l'élimination au moyen d'un procédé classique du groupe protecteur de l'atome d'azote d'un composé correspondant portant un groupe protecteur classique de l'atome d'azote, comprenant pour un composé de formule I dans laquelle R représente G, l'élimination d'un groupe activateur/protecteur Rx d'un composé correspondant de formule Vb : 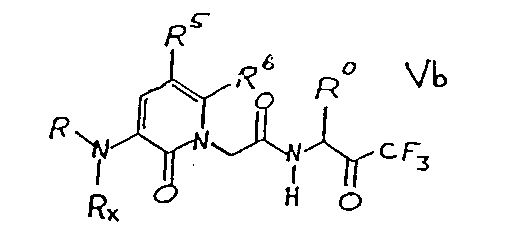
(H) pour un composé de formule I portant un groupement de formule COORa, CH₂COORa, CONRbRc, CH₂CONRbRc, COO(CH₂)₂NReRf ou CONRdSO₂R¹, l'acylation d'un composé correspondant de formule HORa, HNRbRC, HO(CH₂)₂NReRf ou HNRdSO₂R¹ avec un acide correspondant de formule I portant un groupement de formule COORa dans laquelle Ra représente l'hydrogène, ou un des ses dérivés activés ;
(I) pour un composé de formule I portant un groupe acyloxy inférieur ou un groupe de formule NRgCHO, NRgCOR², NRgCOOR², NRhCQNRiRj ou NRkSO₂R³, l'acylation ou la sulfonylation d'un composé correspondant de formule I portant un groupe hydroxy ou un groupe amino de formule NHRg, NHRh ou NHRk (c'est-à-dire un groupe amino de formule NReRf dans laquelle Re représente l'hydrogène et Rf représente un groupe Rg, Rh ou Rk) avec un dérivé activé d'un acide correspondant de formule HOCHO, HOCOR², HOCOOR², HOCQNRiRj (y compris un isocyanate ou isothiocyanate) ou HOSO₂R³, respectivement, en utilisant un procédé classique ;
(J) pour un composé de formule I qui porte un groupe hétéroaryle N-oxyde, l'oxydation d'un composé correspondant de formule I qui porte un groupe hétéroaryle en utilisant un oxydant classique ; ou
(K) pour un composé de formule I qui porte un groupe amino primaire, la réduction d'un composé correspondant portant un groupe nitro en utilisant un procédé de réduction classique ; et
puis, pour n'importe lequel des modes opératoires précités, lorsqu'un sel pharmaceutiquement acceptable d'un composé acide ou basique de formule I est requis, la réaction de la forme acide ou basique d'un tel composé de formule I avec une base ou un acide donnant un ion complémentaire physiologiquement acceptable, ou l'utilisation de n'importe quel autre mode opératoire classique ; et
chacun des groupes R, R⁰, R⁵, R⁶, D, W, G, Ra-Rn, R¹-R³ et Q, à l'exception d'une description plus spécifique, répond à la définition figurant dans l'une quelconque des revendications 1 à 10.

12. Composé de formule II, dans laquelle R, R⁰, R⁵ et R⁶ répondent aux définitions suivant la revendication 1, ou un de ses sels.

13. Composé de formule Vb, dans laquelle R a une valeur définie pour G dans la revendication 1 ; R⁰, R⁵ et R⁶ répondent aux définitions suivant la revendication 1 ; et Rx représente un groupe qui protège et active un groupe amino primaire pour une substitution, ou un de ses sels.

14. Composition pharmaceutique comprenant un composé répondant à la définition suivant la revendication 1, ou un de ses sels pharmaceutiquement acceptables, et un diluant ou support pharmaceutiquement acceptable.
